(19) Europäisches Patentamt
European Patent Office
Office européen des brevets

(11) **EP 3 329 251 B1**

(12) **EUROPÄISCHE PATENTSCHRIFT**

(45) Veröffentlichungstag und Bekanntmachung des Hinweises auf die Patenterteilung:
**23.01.2019  Patentblatt 2019/04**

(21) Anmeldenummer: **16745134.3**

(22) Anmeldetag: **01.08.2016**

(51) Int Cl.:
*G01N 21/27* (2006.01)          *G01J 3/42* (2006.01)
*G01N 21/31* (2006.01)

(86) Internationale Anmeldenummer:
**PCT/EP2016/068344**

(87) Internationale Veröffentlichungsnummer:
**WO 2017/017284 (02.02.2017 Gazette 2017/05)**

(54) **BREITBANDIGE RESONATOR-VERSTÄRKTE OPTISCHE ABSORPTIONSSPEKTROSKOPIE**

BROAD BAND CAVITY ENHANCED OPTICAL ABSORPTION SPECTROSCOPY

SPECTROSCOPIE OPTIQUE D'ABSORPTION À GRANDE LARGEUR DE BANDE AMÉLIORÉ À CAVITÉ

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **30.07.2015  EP 15179088**

(43) Veröffentlichungstag der Anmeldung:
**06.06.2018  Patentblatt 2018/23**

(73) Patentinhaber: **Airyx GmbH**
**69214 Eppelheim (DE)**

(72) Erfinder:
• **PÖHLER, Denis**
**69214 Eppelheim (DE)**
• **HORBANSKI, Martin**
**69126 Heidelberg (DE)**
• **PLATT, Ulrich**
**69221 Dossenheim (DE)**

(74) Vertreter: **Lippert Stachow Patentanwälte**
**Rechtsanwälte**
**Partnerschaft mbB**
**Frankenforster Strasse 135-137**
**51427 Bergisch Gladbach (DE)**

(56) Entgegenhaltungen:
• **D. J. HOCH ET AL: "An instrument for measurements of BrO with LED-based Cavity-Enhanced Differential Optical Absorption Spectroscopy", ATMOSPHERIC MEASUREMENT TECHNIQUES, Bd. 7, Nr. 1, 1. Januar 2014 (2014-01-01), Seiten 199-214, XP055241393, DOI: 10.5194/amt-7-199-2014**
• **U. PLATT ET AL: "Broadband Cavity Enhanced Differential Optical Absorption Spectroscopy (CE-DOAS) - applicability and corrections", ATMOSPHERIC MEASUREMENT TECHNIQUES, Nr. 2, 16. November 2009 (2009-11-16), Seiten 713-723, XP055250019,**
• **University Of Heidelberg: "Advanced Lab Course Cavity-Enhanced-DOAS" In: "Advanced Lab Course Cavity-Enhanced-DOAS", 5. August 2014 (2014-08-05), XP055250297, Seiten 1-74, Seiten 20-23**

EP 3 329 251 B1

## Beschreibung

Die Erfindung betrifft ein Verfahren zum Ermitteln von Konzentrationen von Absorbergasen in einem zu messenden Gasgemisch mittels eines spektroskopischen Messgeräts gemäß dem Oberbegriff von Anspruch 1 sowie ein spektroskopisches Messgerät.

Herkömmlicherweise werden spektroskopische Messgeräte dazu verwendet, Konzentrationen von Absorbergasen in einem zu messenden Gasgemisch zu ermitteln. Beispielsweise kann es sich bei dem zu messenden Gasgemisch um Umgebungsluft handeln, wobei die Konzentrationen von Absorbergasen in der Umgebungsluft bestimmt werden sollen. Mit Absorbergasen sind dabei allgemein gasförmige Stoffe bezeichnet, die jeweils in einem bestimmten Wellenlängenbereich Licht absorbieren. Beispielsweise fallen unter den Begriff "Absorbergase" Spurengase wie etwa NO, $NO_2$, $NO_3$ oder $O_3$. Gerade die Konzentrationen von solchen Spurengasen werden häufig durch entsprechende spektroskopische Messgeräte bestimmt. Das Grundprinzip sämtlicher spektroskopischer Messgeräte beruht darauf, dass jedes Absorbergas aufgrund seiner charakteristischen Molekülstruktur bei Anregung durch Licht eine bestimmte Absorptionsstruktur aufweist. Wird ein Molekül eines solchen Absorbergases durch eine Lichtquelle angeregt, die über eine bestimmte spektrale Breite, d. h. über einen bestimmten Wellenlängenbereich, Licht mit einer wellenlängenabhängigen Lichtintensität ausstrahlt, so absorbiert dieses Molekül gemäß seiner spezifischen Absorptionsstruktur einen Anteil dieses Lichts, wobei der Anteil des absorbierten Lichts von der Wellenlänge abhängt. Die Absorptionsstruktur gibt somit die wellenlängenabhängige Absorptionseigenschaft eines Absorbergases mit Bezug auf die Absorption von Licht an. Wenn von einer Lichtquelle Licht in ein Gasgemisch ausgesandt wird, wobei das Licht in dem Gasgemisch einen Lichtweg zurücklegt, so wird gemäß der spezifischen Absorptionsstruktur eines Absorbergases umso mehr Intensität des Lichts absorbiert, je mehr Moleküle des Spurengases das Licht auf seinem Lichtweg trifft. Die Lichtintensität, die gemessen wird, nachdem von einer Lichtquelle ausgesandtes Licht einen bestimmten Lichtweg in einem bestimmten Gasgemisch mit einem Absorbergas durchlaufen hat, hängt somit sowohl von der Weglänge des Lichtwegs als auch von der Konzentration des Absorbergases in der Umgebungsluft ab. Mathematisch kann dieses Grundprinzip über das Beer-Lambert-Gesetz beschrieben werden: $I = I_0 * \exp[-\sigma * x * L]$, wobei $I$ die Lichtintensität nach Durchlaufen des Lichtwegs in der Umgebungsluft, $I_0$ die von der Lichtquelle in die Umgebungsluft ausgesandte Lichtintensität, $\sigma$ den Absorptionswirkungsquerschnitt des Spurengases, $x$ die Konzentration des Spurengases und $L$ die Weglänge des Lichtwegs angeben. Der Absorptionswirkungsquerschnitt eines Spurengases ist selbstverständlich abhängig von der Wellenlänge und gibt die Absorptionsstruktur eines Spurengases präzise an.

Vor dem Hintergrund dieses Grundprinzips sind herkömmliche spektroskopische Messgeräte stets als eine Anordnung aufgebaut, bei der Licht, das von einer Lichtquelle ausgesandt wird, einen vorbestimmten oder bestimmbaren Lichtweg zurücklegt, wobei am Ende des Lichtwegs ein Detektor angeordnet ist, mit dem eine Lichtintensität gemessen werden kann. Zur Realisierung von spektroskopischen Messgeräten sind sehr unterschiedliche Möglichkeiten bekannt. So existieren beispielsweise spektroskopische Messgeräte, bei denen der Lichtweg und damit auch die Weglänge des Lichtwegs durch geometrische Anordnung von Spiegeln zwischen einem Startpunkt und einem Zielpunkt geometrisch festgelegt ist. Zur Gewährleistung einer hohen Messgenauigkeit sind solche spektroskopische Messgeräte mit einem geometrisch festgelegten Lichtweg mit einer Weglänge von mehreren Kilometern bekannt. Ein gänzlich anderer Ansatz wird bei spektroskopischen Messgeräten beschritten, die eine Messzelle mit einem optischen Resonator aufweisen. Der optische Resonator ist in der Messzelle angeordnet und umfasst eine Spiegelanordnung, die dazu dient, Licht möglichst oft innerhalb der Spiegelanordnung zu reflektieren. Die Weglänge des Lichtwegs ist bei einem solchen Aufbau nicht geometrisch vorgegeben sondern hängt vielmehr beispielsweise von der Reflektivität der Spiegelanordnung und von der Absorption von Licht innerhalb der Messzelle ab. Je höher die Reflektivität der Spiegelanordnung und je geringer die Absorption, desto länger ist der Lichtweg. Die Absorption kann beispielsweise durch Elemente des Gasgemischs, wie beispielsweise die darin enthaltenen Spurengase, und/oder durch das Absorptionsverhalten der Spiegelanordnung bedingt sein. Bei solchen Messgeräten mit optischem Resonator wird mit einem Detektor die Lichtintensität gemessen, die aus der Messzelle ausgekoppelt wird. Das Auskoppeln kann beispielsweise über einen teiltransparenten Strahlteiler in der Messzelle oder über einen teiltransparenten Spiegel der Spiegelanordnung erfolgen. Da der Lichtweg nicht geometrisch vorbekannt ist, sind Kalibrationsmessungen erforderlich, damit aus einer gemessenen Lichtintensität Rückschlüsse auf die Konzentration von Absorbergasen in dem Gasgemisch gezogen werden können, das in der Messzelle angeordnet ist. Über die Kalibrationsmessungen wird ein Wert ermittelt, der die Weglänge des Lichtwegs charakterisiert, beispielsweise eine "mittlere Weglänge", eine "Reflektivität" der Spiegelanordnung oder eine "mittlere Aufenthaltszeit" des Lichts in der Messzelle. Die genannten Möglichkeiten zur Charakterisierung der Weglänge sind äquivalent und ineinander umrechenbar. Im Vergleich zu den beispielhaft beschriebenen Messgeräten mit geometrisch festgelegtem Lichtweg weisen Messgeräte mit optischem Resonator den erheblichen Vorteil auf, dass aufgrund der Vielfachreflexionen in dem optischen Resonator auch mit einer geringen Baugröße des Resonators und damit des gesamten spektroskopischen Messgeräts ein so langer Lichtweg erreicht werden kann, dass eine präzise Messung von Konzentrationen von Absorbergasen in einem Gasgemisch möglich ist. Daher eignen sich solche spektroskopischen Messgeräte gerade auch für lokale, d. h. räumlich aufgelöste Messungen von Absorbergaskonzentrationen und sind darüber hinaus kostengünstig und einfach herstellbar.

Die vorliegende Erfindung betrifft solche beschriebenen spektroskopischen Messgeräte mit optischem Resonator und ein Verfahren zum Ermitteln von Konzentrationen von Absorbergasen in einem Gasgemisch mittels solcher spektroskopischer Messgeräte. Dabei werden die Konzentrationen der Absorbergase ausgehend von den wellenlängenabhängigen Messwerten für die Lichtintensität, die aus dem Detektor ausgelesen werden, bestimmt, indem aus den ausgelesenen wellenlängenabhängigen Messwerten ein wellenlängenabhängiger Verlauf der Lichtintensität als eine wellenlängenabhängige Messwertfunktion dargestellt wird und indem eine theoretische wellenlängenabhängige Funktion nach physikalischen Gesetzen definiert wird, in der die Konzentrationen als wählbare Parameter enthalten sind, wobei die Konzentrationen durch eine Anpassungsrechung zwischen der theoretischen Funktion und der Messwertfunktion ermittelt werden. Die wellenlängenabhängige Messwertfunktion wird dabei aus den aus dem Detektor ausgelesenen Messwerten unmittelbar berechnet und gibt einen nur von der Wellenlänge abhängigen Verlauf eines Zahlenwerts wieder. Beispielsweise kann die wellenlängenabhängige Messwertfunktion unmittelbar als Verlauf der gemessenen Lichtintensität in Abhängigkeit von der Wellenlänge definiert werden. Beispielsweise kann die wellenlängenabhängige Funktion darüber definiert werden, dass die ausgelesenen wellenlängenabhängigen Messwerte für die Lichtintensität jeweils mit einem konstanten Faktor multipliziert oder zu einem konstanten Summanden addiert werden, woraus ein Funktionswert berechnet wird, wobei dieser Funktionswert in Abhängigkeit von der Wellenlänge als wellenlängenabhängige Messwertfunktion dargestellt wird.

Während eine geeignete Messwertfunktion durch sehr einfache Rechnung unmittelbar aus den Messwerten gewonnen werden kann, hat sich die Formulierung einer sinnvollen theoretischen Funktion und die Durchführung einer Anpassungsrechnung als schwierig herausgestellt. Dabei ist zu berücksichtigen, dass während der Anpassungsrechnung stets eine numerische Variation der Konzentrationen als frei wählbare und damit anzupassende Parameter der theoretischen Funktion durchgeführt werden muss, bis die theoretische Funktion in ihrem Verlauf ausreichend gut an die Messwertfunktion angenähert ist. Erst wenn durch entsprechende Wahl der Konzentrationen als anzupassende Parameter der theoretischen Funktion eine ausreichend gute Annäherung zwischen theoretischer Funktion und Messwertfunktion erfolgt ist, kann davon ausgegangen werden, dass die in der Anpassungsrechnung ermittelten Konzentrationen tatsächlich die Konzentrationen der Absorbergase in dem Gasgemisch wiedergeben. Die Beurteilung, wann die theoretische Funktion ausreichend gut an die Messwertfunktion angenähert ist, erfolgt über in der Fehlerrechnung bekannte Methoden, wie beispielsweise über die Bestimmung der Root-Mean-Square-Deviation.

Damit eine solche Anpassungsrechnung überhaupt numerisch durchgeführt werden kann, ist es erforderlich, dass die theoretische Funktion eine so einfache Abhängigkeit von den Konzentrationen als anzupassende Parameter aufweist, dass über numerische Wahl der Konzentrationen eine ausreichend gute Annäherung der theoretischen Funktion an die Messwertfunktion erfolgen kann. Gleichzeitig ist jedoch selbstverständlich erforderlich, dass die theoretische Funktion die physikalisch erwartete Messwertfunktion möglichst exakt gemäß den physikalischen Gesetzen beschreibt. Hierin liegt das entscheidende Problem bei den herkömmlichen Verfahren zur Bestimmung der Konzentrationen der Absorbergase über die spektroskopischen Messgeräte mit optischem Resonator, die die vorliegende Erfindung betrifft.

Denn bei solchen spektroskopischen Messgeräten lässt sich eine physikalisch erwartete Messwertfunktion nur durch sehr komplexe mathematische Funktionen exakt beschreiben. Denn für die Formulierung entsprechender theoretischer Funktionen müssen mehrere solchen spektroskopischen Messgeräten inhärente Randbedingungen berücksichtigt werden. Zum einen hängt die gemessene Lichtintensität von der Konzentration der Absorbergase und der mittleren Weglänge des Lichtwegs ab, den das Licht in der Messzelle zurücklegt. Je länger der Lichtweg bei gleichbleibender Konzentration der Absorbergase ist, desto stärker ist die Absorption in der Messzelle und desto geringer ist die gemessene Lichtintensität. Zum anderen hängt der Lichtweg sowohl von dem Zustand des Messgeräts ab (z. B. Ausrichtung der Spiegelanordnung des Resonators und Reflektivität der Spiegel, d. h. insbesondere auch Verschmutzung der Spiegel) als auch von den Konzentrationen der Absorbergase selbst, da die mittlere Weglänge umso geringer wird, je mehr Licht in der Messzelle absorbiert wird, d. h. je größer die Konzentrationen der Absorbergase sind. Darüber hinaus hängt die gemessene Lichtintensität auch von den Eigenschaften des Detektors ab, die typischerweise durch eine Instrumentenfunktion, die den Detektor und das gesamte spektroskopische Messgerät kennzeichnet, dargestellt werden können. Diese Abhängigkeit von Instrumentenfunktionen des spektroskopischen Messgeräts ist für die physikalisch korrekte Darstellung der theoretischen Funktion umso mehr deswegen relevant, weil sowohl die Absorptionsstrukturen vieler Absorbergase als auch die die mittlere Weglänge bestimmenden Eigenschaften des spektroskopischen Messgeräts (insbesondere die Reflektivität der Spiegel des Resonators) sehr stark wellenlängenabhängig sind. Eine physikalisch korrekte Darstellung der theoretischen Funktion erfordert somit zunächst eine Formulierung der erwarteten Messwertfunktion nach physikalischen Gesetzmäßigkeiten betreffend den Verlauf des Lichts, d. h. der Lichtweg, in der Messzelle und die Absorption und sodann eine Faltung dieser Formulierung mit der Instrumentenfunktion zur Berücksichtigung der Messeigenschaften des Messgeräts, insbesondere des Detektors des Messgerät.

Aufgrund der dargestellten Schwierigkeiten bei der Formulierung einer physikalisch exakten aber dennoch numerisch anpassbaren theoretischen Funktion wurden im Stand der Technik verschiedene Näherungen durchgeführt, um zu einer theoretischen Funktion zu gelangen, über die eine Anpassungsrechnung mit der Messwertfunktion erfolgen kann. Ein gebräuchlicher Ansatz besteht darin, die Messwertfunktion zu definieren als $M(\lambda) = \dfrac{I_0(\lambda)}{I(\lambda)} - 1$ und die

theoretische Funktion zu definieren als $T(\lambda) = L_0(\lambda) * \sum\limits_i \varepsilon_i(\lambda)$. Dabei bezeichnet $I_0(\lambda)$ den wellenlängenabhängigen Verlauf einer Ausgangslichtintensität, $I(\lambda)$ den wellenlängenabhängigen Verlauf der Lichtintensität, der sich aus den Messwerten während der Messung zur Bestimmung der Konzentrationen ergibt, $L_0$ eine Geräteweglänge und $\varepsilon_i$ die verschiedenen Extinktionskoeffizienten der $i$ verschiedenen Absorbergase. $\varepsilon_i$ wird dabei häufig ausgedrückt als $x_i{}^* \sigma_i$, wobei $x_i$ die Konzentration eines bestimmten der $i$ angenommenen Absorbergase und $\sigma_i$ den literaturbekannten Wirkungsquerschnitt des bestimmten Absorbergases angibt. Bei üblichen Verfahren, die diese Näherung anwenden und zur Durchführung der Anpassungsrechnung $M(\lambda)$ mit $T(\lambda)$ gleichsetzen, werden zunächst $I_0(\lambda)$ und $L_0$ in Kalibrationsmessungen bestimmt. $I_0(\lambda)$ bezeichnet dabei die Ausgangslichtintensität, die mit dem Detektor gemessen wird, wenn in der Messzelle "Nullluft" angeordnet ist. Als Nullluft wird üblicherweise möglichst reine Luft verwendet, beispielsweise Umgebungsluft, die durch einen Aerosolfilter zum Entfernen von Streuern und/oder durch weitere Filter zum Herausfiltern von Absorbern gefiltert wurde. Beispielsweise kann als Nullluft auch $N_2$, $O_2$ oder ein $N_2$-$O_2$-Gemisch verwendet werden. Zur Bestimmung von $L_0$ sind unterschiedliche Kalibrationsmessungen und verschiedene Methoden zur Durchführung solcher Kalibrationsmessungen bekannt. Gemäß einer Methode wird als $L_0(\lambda)$ eine mittlere Weglänge des Lichtwegs in der Messzelle bestimmt, indem die Messzelle für eine erste Messung mit Helium geflutet wird und für eine zweite Messung mit Nullluft. Bei beiden Messungen wird die Lichtintensität am Ausgang der Messzelle gemessen. Da davon ausgegangen werden kann, dass die Unterschiede zwischen den bei der ersten und zweiten Messung gemessenen Lichtintensitäten weit überwiegend auf einer unterschiedlichen Rayleigh-Streuung in Luft und Helium beruhen, die jeweils von dem Rayleigh-Streuquerschnitt und damit der Teilchengröße in Luft bzw. Helium abhängt, kann eine mittlere Weg-

$$L_0(\lambda) = \frac{\dfrac{I_{Luft}(\lambda)}{I_{He}(\lambda)} - 1}{\varepsilon_{He}(\lambda) - \varepsilon_{Luft}(\lambda)},$$

länge des Lichtwegs und damit die Geräteweglänge $L_0(\lambda)$ ermittelt werden aus: wobei durch $I_{Luft}$ die gemessene Lichtintensität bei Spülung der Messzelle mit Nullluft, $I_{He}$ die gemessene Lichtintensität bei Spülung der Messzelle mit Helium und $\varepsilon_{He}$ bzw. $\varepsilon_{Luft}$ der Rayleigh-Extinktionskoeffizient angegeben ist, wobei der Extinktionskoeffizient aus $\varepsilon = \sigma^* n$ berechenbar ist, wobei $\sigma$ den in der Literatur bekannten Rayleigh-Streuquerschnitt und $n$ die Teilchenzahldichte angibt, die in guter Näherung über das ideale Gasgesetz bei bekanntem Druck und Temperatur berechenbar ist. Eine andere Methode besteht darin, die Messzelle mit einer Gasmischung zu fluten, die eine vorbekannte Konzentration eines bestimmten Spurengases enthält. Aus der gemessenen Lichtintensität und der bekannten Absorptionsstruktur des Spurengases kann dann unmittelbar Rückschluss auf eine mittlere Weglänge des Lichtwegs gezogen und somit die Geräteweglänge bestimmt werden. Allerdings kann bei dieser Methode nur eine Aussage über die Wellenlängenabhängigkeit der Weglänge des Lichtwegs innerhalb des Wellenlängenbereichs der Absorptionsstruktur des bestimmten Spurengases getroffen werden. Bei der Verwendung von Lasern als Lichtquelle für das spektroskopische Messgerät ist ferner eine weitere Methode für die Kalibrationsmessung bekannt, bei der die Abklingkonstante der Lichtintensität ermittelt wird, nachdem der Laser ein- oder ausgeschaltet wurde. Die Abklingkon-

$$I(t) = I(t_0) * \exp\left[ -\frac{c}{L_0}(t - t_0) \right],$$

stante kann ermittelt werden durch: wobei $I$ die gemessene Lichtintensität, $t_0$ einen bestimmten Zeitpunkt (nach dem Ausschalten), $c$ die Lichtgeschwindigkeit und $L_0$ eine mittlere Weglänge des Lichtwegs, d. h. die Geräteweglänge, angeben. Die Abklingkonstante ist durch $\dfrac{c}{L_0}$ angegeben.

Das beschriebene herkömmliche Verfahren zur Bestimmung von Konzentrationen von Absorbergasen, bei dem die Messwertfunktion $M(\lambda)$ mit der theoretischen Funktion $T(\lambda)$ gleichgesetzt wird unter Definition von

$$\frac{I_0(\lambda)}{I(\lambda)} - 1 = L_0(\lambda) * \sum\limits_i \varepsilon_i(\lambda),$$

kann nur dann zu einer näherungsweise korrekten Bestimmung der Konzentrationen führen, wenn 1.) nur eine relativ geringe Absorption von Licht in der Messzelle auftritt oder die Messwerte mit einem sehr teuren Detektor mit sehr hoher spektralen Auflösung, d.h. Auflösung mit Bezug auf die Wellenlänge, gemessen werden und 2.) das Messgerät bei der Bestimmung von $I(\lambda)$ sich in dem identischen Zustand befindet wie bei der Bestimmung von $I_0(\lambda)$. Denn nur dann sind die mathematischen Näherungen, die bei der Ermittlung der beschriebenen Relation zur Durchführung der Anpassungsrechnung ausgehend von physikalischen Gesetzen durchgeführt wurden, korrekt. Jedenfalls die zweite Bedingung ist jedoch nur durch erheblichen Aufwand realisierbar. Denn zum einen verändern sich die Eigenschaften des optischen Aufbaus des Messgeräts, insbesondere der Messzelle, beispielsweise durch Dejustage oder Verschmutzung der Spiegelanordnung oder Dejustage von lichtlenkenden Linsenanordnungen, so dass die Messungen von $I_0(\lambda)$ und $I(\lambda)$ möglichst unmittelbar hintereinander durchgeführt werden müssen, so dass die Ausgangsmessung von $I_0(\lambda)$ möglichst vor jeder neuen Messung zur Bestimmung von Konzentrationen in einem Gasgemisch durchgeführt werden müssen. Zum anderen variiert bei herkömmlicherweise eingesetzten Lichtquellen die von der Lichtquelle ausgesandte Intensität bereits innerhalb von kurzen Zeitintervallen erheblich. Da jedoch für die Anwendung der genannten Relation zwingende Voraussetzung ist, dass sich das spektroskopische Messgerät und insbesondere auch die Lichtquelle in demselben Zustand befinden, müssen bei herkömmlichen Verfahren zur Bestimmung der Konzentrationen von Absorbergasen aufwendige Maßnahmen ergriffen werden, über die die von der Lichtquelle ausgesandte Intensität stabilisiert werden können. Solche Maßnahmen sind zum einen oft unzureichend und zum anderen kostspielig.

Weiterhin existieren herkömmliche Verfahren zur Ermittlung von Konzentrationen von Absorbergasen, bei

$$M(\lambda) = \ln\left(\frac{I_0(\lambda)}{I(\lambda)}\right)$$

denen die Messwertfunktion $M(\lambda)$ dargestellt wird über und die theoretische Funktion dargestellt

$$T(\lambda) = L_{eff}(\lambda) * \sum_i \varepsilon_i(\lambda) + A \, ,$$

wird über wobei zur Durchführung der Anpassungsrechnung $M(\lambda)$ und $T(\lambda)$ gleichgesetzt werden. Dabei bezeichnen $I_0(\lambda)$, $I(\lambda)$ und $\varepsilon_i(\lambda)$ die oben angegebenen physikalischen Größen. $L_{eff}(\lambda)$ bezeichnet eine "effektive Weglänge", d. h. die mittlere Weglänge, die das Licht in der Messzelle in dem Gasgemisch mit den Absorbern zurücklegt. Während $L_0(\lambda)$ wie oben erläutert die mittlere Weglänge während einer Kalibrationsmessung angibt, gibt $L_{eff}(\lambda)$ die mittlere Weglänge während der Messung zur Ermittlung der Konzentrationen an. Der Summand $A$ ist ein während der Anpassungsrechnung frei wählbarer Parameter, über den Geräteeigenschaften berücksichtigt werden können. Ein Vorteil dieses herkömmlichen Verfahrens im Vergleich zu dem erstgenannten herkömmlichen Verfahren besteht darin, dass eine Variation der von der Lichtquelle ausgesandten Lichtintensität zwischen dem Zeitpunkt der Ausgangsmessung zum Bestimmen der Ausgangslichtintensität und dem Zeitpunkt der eigentlichen Messung zum Bestimmen der Lichtintensität zur Bestimmung der Konzentrationen der Absorbergase nicht unmittelbar zu einer fehlerhaften Anpassungsrechnung führt, da eine Variation um den Faktor $q$ aufgrund der logarithmischen Darstellung von

$$\ln\left(\frac{I_0(\lambda)}{q * I(\lambda)}\right) = \ln\left(\frac{I_0(\lambda)}{I(\lambda)}\right) - \ln q \, .$$

$M(\lambda)$ in dem wählbaren Summanden $A$ "aufgeht", da Allerdings ist bei diesem herkömmlichen Verfahren die Bestimmung der effektiven Weglänge $L_{eff}(\lambda)$, die physikalisch gesehen wie erläutert von den Konzentrationen der Absorbergase selbst abhängt, problematisch. Üblicherweise wird diese effektive Weglänge dadurch angenähert, dass angenommen wird, dass $L_{eff}(\lambda) = L_0(\lambda) * K(\lambda)$, wobei $L_0(\lambda)$ die oben beschriebene

$$K(\lambda) = \frac{\ln\left(\frac{I_0(\lambda)}{I(\lambda)}\right)}{\frac{I_0(\lambda)}{I(\lambda)} - 1} \, .$$

Geräteweglänge darstellt und $K(\lambda)$ einen Korrekturfaktor darstellt, der berechnet wird über Über diese Definition der effektiven Weglänge wird jedoch erneut die Richtigkeit der Anpassungsrechnung abhängig davon, ob der Zustand des spektroskopischen Messgeräts bei der Bestimmung von $I_0(\lambda)$ identisch war mit dem Zustand bei der Bestimmung von $I(\lambda)$, denn nur dann kann die Ermittlung von $K(\lambda)$ für die Bestimmung der effektiven Weglänge $L_{eff}(\lambda)$ als hinreichend korrekt angenommen werden. Prinzipiell wird durch diese üblicherweise angewandte Berechnung von $K(\lambda)$ aus $I_0(\lambda)$ und $I(\lambda)$ das Verfahren mathematisch äquivalent zu der obengenannten Darstellung der Messwer-

$$M(\lambda) = \frac{I_0(\lambda)}{I(\lambda)} - 1 \, ,$$

funktion über wie man durch Einsetzen leicht herleiten kann. Deshalb ist auch bei diesem beschriebenen herkömmlichen Verfahren zur Ermittlung der Konzentrationen von Absorbergasen mittels eines spektroskopischen Messgeräts die Richtigkeit der ermittelten Konzentrationen wesentlich davon abhängig, dass der Zustand des spektroskopischen Messgeräts bei verschiedenen Messungen konstant gehalten wird. Dies ist aufwendig und selbst bei hohem Aufwand kaum vollkommen fehlerfrei realisierbar, so dass auch dieses Verfahren hohe Kosten und gleichzeitig eine hohe Fehleranfälligkeit mit sich bringt. Darüber hinaus ist bei solchen Verfahren selbst bei einer - hypothetischen - tatsächlich perfekten Übereinstimmung des Zustands des Messgeräts während den beiden Messungen zu $I_0(\lambda)$ und $I(\lambda)$ stets die Berechnung von $K(\lambda)$ auf eine Genauigkeit beschränkt, die durch die Eigenschaften des Messgeräts, insbesondere die spektrale Auflösung des Detektors, vorgegeben ist. Beispielsweise sind in der Veröffentlichung "D. J. HOCH ET AL: "An instrument for measurements of BrO with LED-based Cavity-Enhanced Differential Optical Absorption Spectroscopy", ATMOSPHERIC MEASUREMENT ETCHNIQUES, Bd. 7, Nr. 1, 1. Januar 2014 (2014-01-01), Seiten 199-214" Verfahren zum Ermitteln von zumindest einer Konzentration von zumindest einem Absorbergas in einem zu messenden Gasgemisch mittels eines spektroskopischen Messgeräts offenbart. Bei einem dieser Verfahren wird zur Bestimmung der zumindest einen Konzentration eine iterative Anpassungsrechnung durchgeführt, wobei in jedem Iterationsschritt ein DOAS-Fit zwischen der Messwertfunktion und einer theoretischen Funktion durchgeführt wird, wobei die theoretische Funktion als Kalibrationsparameter einen effektiven Wirkungsquerschnitt enthält, der von der optischen Dichte abhängt. In jedem Iterationsschritt wird über den DOAS-Fit die Säulendichte ermittelt, mit der dann ein Wert für die optische Dichte ermittelt wird, der dann zur Bestimmung des effektiven Wirkungsquerschnitts in dem nächsten Iterationsschritt verwendet wird. Nach Abschluss der iterativen Anpassungsrechnung wird aus der Säulendichte die zumindest eine Konzentration ermittelt. Außerdem sind in dem Dokument "U. PLATT ET AL: "Broadband Cavity Enhanced Differential Optical Absorption Spectroscopy (CE-DOAS) - applicability and corrections", ATMOSPHERIC MEASUREMENT TECHNIQUES, Nr. 2, 16. November 2009 (2009-11-16), Seiten 713-723" Grundlagen der CE-DOAS-Spektroskopie erläutert und mathematische Zusammenhänge angegeben, auf Basis derer sich sinnvolle theoretische Funktionen für eine Anpassungsrechnung ermitteln lassen.

Der vorliegenden Erfindung liegt die Aufgabe zugrunde, ein Verfahren zur Ermittlung von Konzentrationen von Absorbergasen in einem zu messenden Gasgemisch mittels eines spektroskopischen Messgeräts bereitzustellen, das eine möglichst einfache, kostengünstige und fehlerfreie Ermittlung der Konzentrationen ermöglicht und das insbesondere die beschriebenen Nachteile herkömmlicher Verfahren behebt. Weiterhin liegt der vorliegenden Erfindung die Aufgabe zugrunde, ein spektroskopisches Messgerät bereitzustellen, das möglichst kostengünstig herstellbar ist und eine möglichst fehlerfreie Ermittlung von Konzentrationen von Absorbergasen in einem zu messenden Gasgemisch gewährleistet.

Als eine Lösung zumindest einer der beschriebenen Aufgaben schlägt die vorliegende Erfindung ein Verfahren mit den Merkmalen von Anspruch 1 vor.

Bei dem erfindungsgemäßen Verfahren wird ein zu messendes Gasgemisch in der Messzelle eines spektroskopischen Messgeräts angeordnet, das die Messzelle, eine Lichtquelle, einen Detektor und eine Recheneinheit umfasst. Die Messzelle weist einen optischen Resonator auf. Dabei dient das erfindungsgemäße Verfahren dem Ermitteln von Konzentrationen von Absorbergasen, die in dem zu messenden Gasgemisch enthalten sind. Hierzu wird mit Hilfe der Lichtquelle ein Lichtstrahl durch einen Eingang der Messzelle in die Messzelle gesandt und mit dem Detektor, der außerhalb der Messzelle an einem Ausgang der Messzelle angeordnet ist, ein wellenlängenabhängiger Messwert für eine Lichtintensität von Licht ermittelt, das aus dem Ausgang austritt. Das Ermitteln eines wellenlängenabhängigen Messwerts erfolgt, indem der Detektor einen Messwert für die Lichtintensität angibt, den er einer bestimmten Wellenlänge zuordnet. Dabei ist die Lichtquelle zum Aussenden von Licht mit unterschiedlicher Wellenlänge ausgebildet. Beispielsweise kann die Lichtquelle als spektral breitbandige Lichtquelle ausgebildet sein, die Licht in einem Wellenlängenbereich von mindestens 100 pm, insbesondere von mindestens 1 nm, insbesondere mindestens 10 nm ausstrahlt. Beispielsweise kann die Lichtquelle als durchstimmbarer Laser ausgebildet sein, über den Licht in unterschiedlichen Wellenlängenbereichen ausgestrahlt werden kann.

Aus den aus dem Detektor ausgelesenen und somit ermittelten Messwerten wird ein wellenlängenabhängiger Verlauf der Lichtintensität als eine wellenlängenabhängige Messwertfunktion dargestellt. Beispielsweise kann die Messwertfunktion direkt aus den Messwerten als $I(\lambda)$ dargestellt werden, indem die Messwerte $I_n$, die jeweils bestimmten Wellenlängen $\lambda_n$ zugeordnet sind, unmittelbar als Verlauf $I(\lambda)$ dargestellt werden. Beispielsweise kann die Messwertfunktion dargestellt werden über $\dfrac{I(\lambda)}{I_0(\lambda)}$ oder $\ln\left(\dfrac{I(\lambda)}{I_0(\lambda)}\right)$, wobei $I_0$ den wellenlängenabhängigen Verlauf einer Ausgangslichtintensität wiedergibt, die über das spektroskopische Messgerät durch Auslesen des Detektors während einer Ausgangsmessung, insbesondere einer Kalibrationsmessung, während der Nullluft bekannten Eigenschaften in der Messzelle angeordnet war, ermittelt wurde. Beispielsweise kann die Messwertfunktion allgemein darüber ermittelt werden, dass der gemessene Verlauf $I(\lambda)$ mit einem Zahlenwert, der von der Wellenlänge $\lambda$ abhängen kann, multipliziert wird oder zu einem solchen Zahlenwert addiert wird. In jedem Fall gibt die Messwertfunktion den wellenlängenabhängigen Verlauf der über den Detektor gemessenen Lichtintensität wieder.

Ferner wird bei dem erfindungsgemäßen Verfahren eine theoretische wellenlängenabhängige Funktion definiert, indem der theoretisch erwartete Verlauf der definierten Messwertfunktion ausgehend von physikalischen Gesetzen mathematisch formuliert wird. Dabei werden in der theoretischen Funktion die Konzentrationen der Absorbergase als frei wählbare Parameter dargestellt. Dabei wird je nach Anwendungsfall und hierzu vorgesehener Ausführungsform von einer bestimmten Anzahl an Absorbergasen ausgegangen. Es versteht sich von selbst, dass in einer besonders einfachen Ausführungsform das Verfahren zur Bestimmung von der Konzentration von nur einem Absorbergas durchgeführt wird, so dass dann in der theoretischen Funktion nur eine Konzentration von nur einem Absorbergas enthalten ist. Bei der nachfolgenden Darstellung, insbesondere auch mit Bezug auf vorteilhafte erfindungsgemäße Ausführungsformen, wird stets allgemein auf "Absorbergase" Bezug genommen, also auf eine bestimmte Menge an Absorbergasen, die selbstverständlich erfindungsgemäß auch auf nur ein Absorbergas beschränkt sein kann, wodurch sich die jeweilige vorteilhafte erfindungsgemäße Ausführungsform entsprechend vereinfacht. Für den Fachmann ist selbstverständlich, dass entsprechend vereinfachte erfindungsgemäße Ausführungformen beispielsweise bei sehr einfach zusammengesetzten Gasgemischen oder bei einer Lichtquelle mit einem sehr beschränkten Spektrum zum Einsatz kommen können. Die theoretische Funktion ist neben den als Parameter dargestellten Konzentrationen zumindest auch noch von einem wellenlängenabhängigen Kalibrationsparameter abhängig, über den der Zustand des Messgeräts während der Messung zur Ermittlung der Messwerte zur Bestimmung der Messwertfunktion berücksichtigt wird. Die Definition der theoretischen Funktion in Abhängigkeit von einem solchen Kalibrationsparameter ist aus den obengenannten Gründen wegen des prinzipiellen konstruktiven Aufbaus des spektroskopischen Messgeräts mit optischem Resonator zwingend erforderlich. Bei dem erfindungsgemäßen Verfahren erfolgt die Anpassungsrechnung zwischen der theoretischen Funktion und der Messwertfunktion, indem zumindest die Konzentrationen der Absorbergase, die in der theoretischen Funktion als wählbare Parameter enthalten sind, angepasst werden, um die theoretische Funktion in ihrem Verlauf an die Messwertfunktion möglichst gut anzunähern. Die theoretische Funktion kann neben den Konzentrationen weitere anzupassende Fit-Parameter, beispielsweise weitere Extinktionsparameter, enthalten, in denen physikalische Gegebenheiten während der Ermittlung der Messwerte, d. h. während der Messung, berücksichtigt werden. Solche weiteren Fit-Parameter sind von den Konzentrationen der Absorbergase unabhängig und können in der theoretischen Funktion berücksichtigt werden, damit über die Anpassungsrechnung eine möglichst korrekte und somit möglichst fehlerfreie Annäherung der theoretischen Funktion an die Messwertfunktion erfolgen kann.

Erfindungsgemäß wird zur Durchführung der Anpassungsrechnung der Kalibrationsparameter als Funktion von einem vorbestimmten wellenlängenabhängigen Geräteparameter und einem wellenlängenabhängigen Korrekturfaktor definiert, wobei der Korrekturfaktor als Funktion von den Konzentrationen definiert wird. Wie oben erläutert kann bei einer besonders einfachen Ausführungsform der Korrekturfaktor als Funktion von nur einer Konzentration von nur einem Absorbergas definiert werden. Über diese funktionale Darstellung des Kalibrationsparameters können die Eigenschaften des spektroskopischen Messgeräts, insbesondere die Einflüsse der Messzelle des Messgeräts auf die Messwerte für die Lichtintensität gleichzeitig besonders einfach und präzise berücksichtigt werden. Dabei wird über den Kalibrationsparameter erfindungsgemäß sowohl der Zustand des Messgeräts, wie beispielsweise die Reflektivität und den Abstand der Spiegel der Spiegelanordnung, als auch die Verkürzung des Lichtwegs aufgrund von Extinktion in dem Gasgemisch berücksichtigt. Der Geräteparameter kann aus einer vorab einmal durchgeführten Kalibrationsmessung erhalten werden. Der Geräteparameter charakterisiert eine Weglänge eines Lichtwegs, den der Lichtstrahl in der Messzelle zurücklegt. Beispielsweise kann als Geräteparameter eine Geräteweglänge wie oben erläutert, insbesondere durch eine Kalibrationsmessung, bestimmt werden, beispielsweise kann als Geräteparameter eine mittlere Aufenthaltszeit oder eine Reflektivität der Messzelle bestimmt werden. Über den Korrekturfaktor wird die durch Extinktion in der Messzelle bedingte Veränderung der Ausbreitung des Lichts in der Messzelle berücksichtigt. Der Korrekturfaktor kann neben den Konzentrationen der Absorbergase auch zumindest einen, insbesondere mehrere weiteren Extinktionsparameter enthalten, über den bzw. die andere Extinktionseffekte berücksichtigt werden, beispielsweise Extinktionseffekte aufgrund von Mie-Streuern, wie beispielsweise Aerosolen, und/oder Extinktionseffekte von Rayleigh-Streuern, wie beispielsweise $N_2$. Besonders bevorzugt wird der Korrekturfaktor als Funktion definiert, die zumindest von den literaturbekannten Wirkungsquerschnitten und von den Konzentrationen der Absorbergase als wählbare Parameter abhängt. Besonders bevorzugt wird der Kalibrationsparameter als Produkt von dem Geräteparameter mit dem Korrekturfaktor definiert, wodurch eine mathematisch möglichst einfache und gleichzeitig physikalisch möglichst korrekte Formulierung des Kalibrationsparameters erfolgen kann.

Erfindungsgemäß wird bei dem Verfahren ein Zyklus umfassend eine Abfolge von Schritten mehrfach hintereinander durchgeführt, wobei in einem ersten Schritt der Abfolge ein Zahlenwert für den Korrekturfaktor aus festgelegten Annahmewerten der Konzentrationen über die den Korrekturfaktor definierende Funktion berechnet wird, in einem zweiten Schritt der Abfolge die theoretische Funktion bestimmt wird, wobei der Kalibrationsparameter aus dem in dem ersten Schritt berechneten Zahlenwert für den Korrekturfaktor berechnet wird, in einem dritten Schritt der Abfolge durch eine Ausgleichsrechnung zwischen der in dem zweiten Schritt bestimmten theoretischen Funktion und der Messwertfunktion Werte für die Konzentrationen ermittelt werden und als neue Annahmewerte festgelegt werden, wobei die in dem dritten Schritt des letzten Zyklus ermittelten Annahmewerte als gemessene Werte der Konzentrationen ausgegeben werden. Als Zahlenwert für den Korrekturfaktor wird in dem ersten Schritt bevorzugt ein von der Wellenlänge hoch aufgelöst abhängiger Zahlenwert vorgesehen. Wie erläutert kann in einer besonders einfachen Ausführungsform, bei der die Konzentration von nur einem Absorbergas bestimmt wird, in dem ersten Schritt der Korrekturfaktor aus einem festgelegten Annahmewert für diese Konzentration berechnet werden, in dem dritten Schritt ein neuer Annahmewert für diese Konzentration ermittelt werden und der in dem dritten Schritt des letzten Zyklus ermittelte Annahmewert für diese Konzentration als der gemessene Wert für diese Konzentration ausgegeben werden.

Das erfindungsgemäße Verfahren ermöglicht die Durchführung einer Anpassungsrechnung bei Zugrundelegung einer mathematisch sehr komplexen und physikalisch sehr exakten theoretischen Funktion. In dieser theoretischen Funktion sind die Konzentrationen zum einen in der den Korrekturfaktor definierenden Funktion enthalten, so dass der Kalibrationsparameter zur Berücksichtigung des Verlaufs bzw. Lichtwegs des Lichts in der Messzelle während der Messung in Abhängigkeit von den Konzentrationen definiert ist. Zum anderen sind die Konzentrationen in einem weiteren, von dem Kalibrationsparameter unabhängigen Funktionsteil der theoretischen Funktion enthalten, über den das Absorptionsverhalten des Gasgemischs, insbesondere das das Absorptionsverhalten umfassende Extinktionsverhalten des Gasgemischs, physikalisch-mathematisch beschrieben wird. Die theoretische Funktion ermöglicht somit eine außergewöhnlich korrekte theoretische Darstellung der zu erwartenden Messwertfunktion. Im Stand der Technik wird eine solche theoretische Funktion zur Durchführung einer Anpassungsrechnung nicht verwendet, da eine direkte Anpassungsrechnung zwischen der Messwertfunktion und der theoretischen Funktion durch unmittelbares numerisches Verändern der Konzentrationen nicht möglich ist, da sich die funktionale Abhängigkeit der Weglänge des Lichtwegs von den Konzentrationen zu sehr von der funktionalen Abhängigkeit des das Absorptionsverhalten des Gasgemischs beschreibenden Funktionsteils von den Konzentrationen unterscheidet. Die vorliegende Erfindung geht daher einen anderen Weg: Die Anpassungsrechnung wird in iterativ durchgeführten Zyklen durchgeführt, wobei vor Beginn eines jeden Zyklus zunächst die Konzentrationen jeweils auf einen Annahmewert festgelegt werden, der ein Zahlenwert ist und somit kein frei wählbarer Parameter, wonach dann in einem ersten Schritt eines jeden Zyklus ein Zahlenwert für den Korrekturfaktor berechnet wird durch Einsetzen der festgelegten Annahmewerte für die Konzentrationen in die den Korrekturfaktor definierende Funktion. In einigen Ausführungsbeispielen kann der Korrekturfaktor nicht nur als Funktion von den Konzentrationen der Absorbergase sondern auch als Funktion von zumindest einem weiteren Extinktionsparameter dargestellt werden, um weitere Extinktionseffekte in der Messzelle zu berücksichtigen, die nicht von den Absorbergasen herrühren. Denn die Extinktion von Licht unter Verkürzung des Lichtwegs, den das Licht in der Messzelle zurücklegt, bevor es aus der Messzelle austritt und auf den Detektor trifft, kann beispielsweise nicht nur durch Absorption sondern auch durch Streuung bedingt sein. Bei diesen Ausführungsformen werden ebenfalls in dem ersten Schritt eines jeden Zyklus Annahmewerte für diesen zumindest einen weiteren in der den Korrekturfaktor definierenden Funktion enthaltenen Extinktionsparameter als Zahlenwerte festgelegt. Der zweite Schritt kann beispielsweise zeitlich nach dem ersten Schritt durchgeführt werden. Der zweite Schritt kann beispielsweise gleichzeitig mit dem ersten Schritt durchgeführt werden, indem beispielsweise die festgelegten Annahmewerte unmittelbar in die theoretische Funktion eingesetzt werden, in der der Korrekturfaktor enthalten ist. Wesentlich ist stets, dass bei der Bestimmung der theoretischen Funktion, die in dem zweiten Schritt erfolgt, der in der theoretischen Funktion enthaltene Korrekturfaktor nicht von den Konzentrationen als anzupassende Parameter abhängt, sondern die festgelegten Annahmewerte als Konzentrationen in den Korrekturfaktor bzw. in die den Korrekturfaktor definierende Funktion eingesetzt sind.

Im Vergleich zu herkömmlichen Verfahren, von denen oben zwei Verfahren beispielhaft skizziert sind, ermöglicht das erfindungsgemäße Verfahren eine sehr exakte Berücksichtigung der Veränderung des Lichtwegs in der Messzelle, die auf Extinktionen in der Messzelle beruht. Die Erfinder haben erkannt, dass die herkömmlichen Verfahren die Verkürzung des Lichtwegs durch Extinktionen nur im Rahmen der Messgenauigkeit des Messgeräts, insbesondere nur im Rahmen der typischerweise niedrigen spektralen Auflösung des Detektors, berücksichtigen. Besonders deutlich wird dies bei dem oben erläuterten Verfahren aus dem Stand der Technik, bei dem eine "effektive Weglänge" $L_{eff}(\lambda)$ ermittelt

$$K(\lambda) = \frac{\ln\left(\frac{I_0(\lambda)}{I(\lambda)}\right)}{\frac{I_0(\lambda)}{I(\lambda)} - 1} \quad .$$

wird über $L_{eff}(\lambda) = L_0(\lambda) * K(\lambda)$ mit                    Da $K(\lambda)$ von $I(\lambda)$ und $I_0(\lambda)$ abhängt, vorliegend sogar nichtlinear abhängt, wird die Bestimmung von $L_{eff}(\lambda)$ zusätzlich verfälscht, falls in der Messzelle ein Gasgemisch mit Absorbergasen enthalten ist, die eine schmalbandige Absorptionsstruktur aufweisen, deren Halbwertsbreite kleiner oder gleich der spektralen Auflösung des Detektors ist. Denn der Detektor glättet dann wegen seiner zu niedrige Auflösung bei der Messung von $I(\lambda)$ bzw. $I_0(\lambda)$ schmalbandige Absorptionen, wodurch diese schwächer erscheinen, was wiederum zu einem verfälschten Korrekturfaktor $K(\lambda)$ führt. Das erfindungsgemäße Verfahren bietet den herkömmlichen Verfahren gegenüber den großen Vorteil, dass eine sehr genaue Berechnung des Korrekturfaktors bei hoher spektraler Auflösung erfolgen kann, die insbesondere nicht durch die spektrale Auflösung des Detektors beschränkt ist. Denn indem der Korrekturfaktor als Funktion von den Konzentrationen mathematischphysikalisch formuliert wird, kann der Korrekturfaktor mit einer sehr hohen spektralen Auflösung definiert werden, beispielsweise über Einführung der literaturbekannten, spektral hochaufgelösten Wirkungsquerschnitte in die den Korrekturfaktor definierende Funktion. Hohe spektrale Auflösung bedeutet hierbei insbesondere eine spektrale Auflösung, die kleiner als 0.5, bevorzugt aber kleiner als 0.1 mal der Halbwertsbreite der schmalbandigen Absorptionsstrukturen des Absorbers ist. Das erfindungsgemäße Verfahren hat damit gegenüber herkömmlichen Verfahren den weiteren Vorteil, dass der Korrekturfaktor auch dann sehr korrekt bestimmt werden kann, wenn das Gasgemisch in der Messzelle Absorbergase mit schmalbandigen Absorptionsstrukturen aufweist, deren Halbwertsbreite kleiner oder gleich der Auflösung des Detektors ist. Ganz prinzipiell unterschätzen in einem solchen Fall die üblichen Verfahren den Korrekturfaktor erheblich. Insbesondere kann das erfindungsgemäße Verfahren eine möglichst schnelle und möglichst wenig Rechenleistung erfordernde aber gleichzeitig möglichst präzise Ermittlung von Konzentrationen von Absorbergasen mit einem Messgerät ermöglichen. Darüber hinaus kann das erfindungsgemäße Verfahren über geeignete Wahl der Messwertfunktion und der theoretischen Funktion ermöglichen, dass die Bestimmung von Konzentrationen von Absorbergasen nicht von einer Variation des Betriebszustands der Lichtquelle (z.B. Variation der emittierten Lichtintensität) beeinflusst ist, beispielsweise durch Wahl der Messwertfunktion M über

$$M(\lambda) = \ln\left(\frac{I_0(\lambda)}{I(\lambda)}\right) \quad,$$

siehe obige Ausführungen. Denn das erfindungsgemäße Verfahren ermöglicht durch die iterative Durchführung der Bestimmung der Konzentrationen bei der Anpassungsrechnung inhärent eine präzise Charakterisierung der Weglänge des Lichtwegs des Lichtstrahls in der Messzelle. Dadurch kann auch bei der Wahl einer entsprechenden Messwertfunktion und einer entsprechenden, insbesondere einer von einer absoluten Intensität unabhängigen, Definition einer theoretischen Funktion eine präzise Bestimmung der Konzentrationen der Absorbergase ermöglicht sein.

In dem ersten Schritt wird somit erfindungsgemäß ein Zahlenwert für den Korrekturfaktor bestimmt. In dem zweiten Schritt eines jeden Zyklus wird aus diesem Zahlenwert des Korrekturfaktors der Kalibrationsparameter berechnet, wobei hierfür ein für den Geräteparameter in einer Kalibrationsmessung zuvor ermittelter Zahlenwert eingesetzt wird. Der in dem zweiten Schritt eines jeden Zyklus ermittelte Kalibrationsparameter ist somit nicht in Abhängigkeit von den Konzentrationen als frei wählbare, anzupassende Parameter definiert, da für die Konzentrationen zur Bestimmung des Kalibrationsparameters die festgelegten Annahmewerte als Zahlenwerte eingesetzt werden. Der Kalibrationsparameter kann in einem Ausführungsbeispiel in dem zweiten Schritt als ein Zahlenwert bestimmt werden. Eine "Berechnung" gemäß der vorliegenden Beschreibung, beispielsweise die Berechnung des Korrekturfaktors aus den Annahmewerten während des ersten Schritts und/oder die Berechnung des Kalibrationsparameters aus dem Korrekturfaktor während des zweiten Schritts, erfolgt allgemein bevorzugt durch einen Rechner. Beispielsweise kann die Berechnung durch analytische oder numerische Rechenverfahren auf Basis der den Korrekturfaktor bzw. den Kalibrationsparameter definierenden Funktion erfolgen. Beispielsweise kann die Berechnung dadurch erfolgen, dass in einer Speicherablage des Rechners vorab einzusetzende Werte und hieraus mittels einer Funktion berechnete Werte abgelegt werden, so dass bei der Durchführung des Verfahrens zur Bestimmung der Konzentration in dem Rechner ausgehend von dem einzusetzenden Wert aus der Speicherablage der dem einzusetzenden Wert zugeordnete, über die Funktion vorab berechnete und in der Speicherablage abgelegte Wert entnommen wird. So können beispielsweise in dem Rechner zu verschiedenen Annahmewerten verschiedene zugeordnete Korrekturfaktoren bzw. zu verschiedenen Korrekturfaktoren verschiedene zugeordnete Werte für den Kalibrationsparameter in einer solchen Speicherablage abgelegt werden, die vorab auf Basis der den Korrekturfaktor bzw. den Kalibrationsparameter definierenden Funktion ermittelt wurden, wobei in dem Rechner in dem ersten Schritt unter Berücksichtigung der festgelegten Annahmewerte der zugeordnete Korrekturfaktor bzw. in dem zweiten Schritt unter Berücksichtigung des berechneten Korrekturfaktors der zugeordnete Kalibrationsparameter aus der Speicherablage entnommen wird. In einem Ausführungsbeispiel enthält der Kalibrationsparameter neben einem Zahlenwert zumindest einen weiteren Ausgleichsparameter, in dem physikalische Effekte zusammengefasst werden, um die Ausgleichsrechnung in dem dritten Schritt eines jeden Zyklus zu vereinfachen.

In dem dritten Schritt eines jeden Zyklus wird dann durch einen Abgleich zwischen der dem in dem zweiten Schritt ermittelten theoretischen Funktion und der Messwertfunktion eine Ausgleichsrechnung durchgeführt, wobei hierzu die in der theoretischen Funktion als frei wählbare Parameter enthaltenen Konzentrationen angepasst werden, um die theoretische Funktion, die in dem zweiten Schritt ermittelt wurde, möglichst nah an die Messwertfunktion anzupassen. Dabei werden für die Ausgleichsrechnung in der Fehlermessung bekannte Methoden angewandt, um den verbleibenden Fehler zwischen der theoretischen Funktion und der Messwertfunktion durch numerisches Anpassen der Konzentrationen möglichst zu verringern. In einem Ausführungsbeispiel, in dem der die Absorptionseigenschaft des Gasgemischs beschreibende Funktionsteil neben den Konzentrationen der Absorbergase weitere Extinktionsparameter zur Charakterisierung des Gasgemischs enthält, können in dem dritten Schritt neben den Konzentrationen auch diese Extinktionsparameter angepasst werden, um die theoretische Funktion möglichst gut an die Messwertfunktion anzunähern. In dem dritten Schritt werden die für die Konzentrationen nach Abschluss der Ausgleichsrechnung ausgegebenen Werte als neue Annahmewerte festgelegt. In Ausführungsformen, in denen die theoretische Funktion zumindest einen weiteren Extinktionsparameter als anzupassende weitere Fit-Parameter enthält, werden auch für die weiteren Extinktionsparameter in dem dritten Schritt neue Annahmewerte festgelegt. Sodann wird der Zyklus bei einer nachfolgenden Iteration wiederholt, wobei die in dem dritten Schritt des zuvor durchgeführten Zyklus ermittelten Annahmewerte für die Konzentrationen und insbesondere für die weiteren Extinktionsparameter als festgelegte Annahmewerte verwendet werden. In dem nachfolgenden Zyklus werden somit zur Ermittlung eines Zahlenwerts für den Korrekturfaktor in dem ersten Schritt Annahmewerte eingesetzt, die über die Ausgleichsrechnung des vorangehenden Zyklus ermittelt wurden. Es kann daher davon ausgegangen werden, dass diese Annahmewerte des nachfolgenden Zyklus näher an den tatsächlichen Werten der Konzentrationen in dem Gasgemisch liegen als die Annahmewerte des vorangehenden Zyklus. Durch die iterative Wiederholung des Zyklus wird eine schrittweise Annäherung der in der Ausgleichsrechnung des jeweiligen Zyklus ermittelten Annahmewerte der Konzentrationen an die tatsächlichen Konzentrationswerte in dem Gasgemisch erreicht.

In einigen Ausführungsformen wird gleichzeitig durch die iterative Wiederholung des Zyklus eine schrittweise Annäherung der Werte der Extinktionsparameter an die tatsächlichen Werte im Gasgemisch erreicht. Dadurch, dass während der Ausgleichsrechnung nicht die funktionale Abhängigkeit des Korrekturfaktors von den Konzentrationen und insbesondere von weiteren Extinktionsparametern als anzupassende Parameter berücksichtigt wird, vermeidet das erfindungsgemäße Verfahren einen numerisch faktisch nicht durchführbaren Abgleich zwischen einer zu komplexen theoretischen Funktion und der Messwertfunktion. Vielmehr ermöglicht das erfindungsgemäße Verfahren eine schrittweise Annäherung der Annahmewerte für die Konzentrationen an die tatsächlichen Konzentrationswerte in dem Gasgemisch, wodurch eine jede Ausgleichsrechnung numerisch durchführbar wird und die gesamte Anpassungsrechnung unter Zugrundelegung einer physikalisch sehr exakt formulierten theoretischen Funktion erfolgen kann.

Besonders bevorzugt werden während der Ausgleichsrechnung eines jeden Zyklus die Konzentrationen und insbesondere weitere Extinktionsparameter als Parameter der theoretischen Funktion verändert zum Verringern der Unterschiede zwischen der theoretischen Funktion und der Messwertfunktion. Durch die fortlaufende Veränderung der Konzentrationen und insbesondere der weiteren Extinktionsparameter der theoretischen Funktion bei jedem Zyklus wird eine fortlaufende Optimierung der Anpassung der theoretischen Funktion an die Messwertfunktion gewährleistet, was mit einer von Zyklus zu Zyklus fortlaufend verbesserten Annäherung der in der jeweiligen Ausgleichsrechnung des jeweiligen Zyklus ermittelten Konzentrationen an die tatsächlich in dem Gasgemisch vorherrschenden Konzentrationen der Absorbergase einhergeht. Dabei ist zu berücksichtigen, dass durch das fortlaufende Verändern sowohl von den weiteren Extinktionsparametern als auch von den Konzentrationen eine möglichst umfassende Anpassung der komplex formulierten theoretischen Funktion erfolgen kann, wodurch eine möglichst fehlerfreie Ermittlung der Konzentrationen gewährleistet werden kann.

In einer Ausführungsform wird der Geräteparameter durch eine Kalibrationsmessung mit dem Messgerät ermittelt, wobei die Ermittlung der gemessenen Werte der Konzentrationen von Absorbergasen durch die Anpassungsrechnung auf Basis von Messwerten erfolgt, die in einer Messung ermittelt werden, die getrennt von der Kalibrationsmessung durchgeführt wird. Die Kalibrationsmessung wird somit mit demselben spektroskopischen Messgerät durchgeführt, mit dem auch die Messung zur Ermittlung der Messwerte zur Bestimmung der Konzentrationen der Absorbergase durch die Anpassungsrechnung durchgeführt wird. Die Kalibrationsmessung kann zeitlich vor oder nach der Messung zur Ermittlung der genannten Messwerte erfolgen. Die Kalibrationsmessung kann beispielsweise durchgeführt werden, während Nullluft in der Messzelle angeordnet wird, beispielsweise Luft, die durch Aerosolfilter und/oder andere Filter, die dem Herausfiltern von Absorbergasen dienen, gefiltert wurde, beispielsweise Nullluft in Form von $N_2$, $O_2$ oder einem $N_2O_2$-Gemisch. In jedem Fall dient die Kalibrationsmessung dazu, einen Zahlenwert für den Geräteparameter zu ermitteln, der bei dem erfindungsgemäßen Durchführen der Anpassungsrechnung zum Ermitteln der Konzentrationen aus den während der Messung ermittelten Messwerten in die theoretische Funktion eingesetzt wird.

In einer Ausführungsform wird die theoretische Funktion definiert als Summe aus einem ersten Summanden, der von dem Kalibrationsparameter und einem Funktionsteil abhängt, der physikalisch-mathematisch ausschließlich schmalbandige Absorptionseigenschaften des Gasgemischs beschreibt und in Abhängigkeit von den Konzentrationen als anzupassende Parameter definiert ist, und einem zweiten Summanden, der als von den Konzentrationen und von dem Kalibrationsparameter unabhängiger Breitbandparameter definiert ist. Diese Ausführungsform ermöglicht eine besonders rechenarme und somit schnelle und kostengünstige Ermittlung der Konzentrationen von Absorbergasen im Gasgemisch. In bestimmten Ausführungsformen werden dabei nur Konzentrationen von solchen Absorbergasen ermittelt, die eine schmalbandige Absorptionsstruktur aufweisen, was jedoch für eine Vielzahl an relevanten Spurengasen zutrifft. Eine schmalbandige Absorptionsstruktur kann beispielsweise darüber definiert werden, dass bei einer Aufnahme der Absorptionsstruktur über eine Messung der Lichtintensität die gewonnenen wellenlängenabhängigen Messwerte nicht mit einem Polynom $\leq$ 3. Ordnung angenähert werden können, ohne dass der Fehler zwischen diesem Polynom und dem wellenlängenabhängigen Verlauf der Messwerte größer ist als der Messfehler während der Messung. Die beschriebene Ausführungsform ermöglicht es, den die Absorptionseigenschaft des Gasgemischs beschreibenden Funktionsteil der theoretischen Funktion nur unter Berücksichtigung solcher physikalischer Gesetzmäßigkeiten mathematisch zu formulieren, die eine schmalbandige Absorption beschreiben. Dadurch kann die theoretische Funktion einfacher formuliert werden, was eine entsprechend einfachere Ausgleichsrechnung mit sich bringt. Besonders bevorzugt wird der Kalibrationsparameter, der in dem ersten Summanden enthalten ist, als Funktion von den Konzentrationen der schmalbandigen Absorbergase und als Funktion von weiteren Extinktionsparametern definiert, über die auch eine breitbandige Extinktion berücksichtig wird.

Dadurch kann eine immer noch einfache, aber noch präzisere Ermittlung der Konzentrationen der schmalbandigen Absorbergase ermöglicht sein, da über die Berücksichtigung auch breitbandiger Extinktionseffekte in dem Kalibrationsparamter der hierdurch bedingten Veränderung des Lichtwegs in der Messzelle Rechnung getragen wird.

Bei einer Realisierung dieser Ausführungsform wird der Breitbandparameter unabhängig von der Anpassungsrechnung aus der Messwertfunktion selbst ermittelt und sodann während der Anpassungsrechnung in die theoretische Funktion eingesetzt. Beispielsweise kann eine solche Ermittlung des Breitbandparameters aus der Messwertfunktion darüber erfolgen, dass eine Glättung der Messwertfunktion berechnet wird, indem jeweils ein Mittelwert aus jeweils zwei Werten der Messwertfunktion gebildet wird, die zwei Wellenlängen zugeordnet sind, die um einen festgelegten Wellenlängenabstand voneinander beabstandet sind. Als ein solcher festgelegter Wellenlängenabstand kann beispielsweise 1 nm, beispielsweise 5 nm gewählt werden. Die Glättung wird aus der Messwertfunktion dadurch berechnet, dass stets der Wert der Glättung in dem gesamten Wellenlängenbereich zwischen den beiden den zwei Werten der Messwertfunktion zugeordneten Wellenlängen auf den Mittelwert festgelegt wird. Alternativ kann der Breitbandparameter beispielsweise aus der Messwertfunktion aus einer vor der Anpassungsrechnung durchgeführten Fitrechnung ermittelt werden. Bei einer solchen Fitrechnung wird die Messwertfunktion zunächst mit einem Polynom 3. Grades (d. h. ein

$$P = \sum_{i=0}^{3} v_i * \lambda^i ,$$

Polynom mit den anzupassenden Parametern $v_i$) angenähert durch Anpassung der Polynomparameter $v_i$. Indem der Breitbandparameter in der Anpassungsrechnung als konstanter Zahlenwert eingesetzt werden kann, kann dann die Ausgleichsrechnung besonders einfach durchgeführt werden. In einer anderen Realisierung des genannten Ausführungsbeispiels wird der Breitbandparameter in die theoretische Funktion eingeführt, indem er als Polynom

$$( P = \sum_{i=0}^{N} v_i * \lambda^i )$$

N-ten Grades in der theoretischen Funktion formuliert wird, wobei die Parameter $v_i$ während der Ausgleichsrechnung mit angepasst werden. Dabei wird $N \leq 5$, insbesondere $\leq 3$ gewählt, da hierüber Breitbandeffekte ausreichend gut angenähert werden können und die Zahl der Polynomparameter $v_i$ dadurch geringgehalten wird, was die Durchführung der Ausgleichsrechnung vereinfacht. Außerdem kann durch die Wahl eines entsprechend geringen Grades des Polynoms sichergestellt sein, dass das Polynom keinen schmalbandigen Extinktionseffekt sondern ausschließlich breitbandige Extinktionseffekte beschreibt. Dadurch kann sichergestellt sein, dass die Bestimmung der Konzentrationen während der Anpassungsrechnung durch die Einführung des Breitbandparameters nicht verfälscht wird.

In einer Ausführungsform wird der Kalibrationsparameter bei der physikalisch-mathematischen Formulierung der theoretischen Funktion so gewählt, dass er eine mittlere Weglänge eines Lichtwegs des Lichts in der Messzelle charakterisiert, wobei die theoretische Funktion definiert wird auf Basis einer Darstellung der Lichtintensität $I(\lambda)$ als $I(\lambda)=I_0(\lambda)\cdot exp(-L.\varepsilon)$, wobei $I_0(\lambda)$ die Lichtintensität des austretenden Lichts bei Anordnung eines Gasgemischs ohne oder mit bekannter Extinktionseigenschaft in der Messzelle angibt, $L$ den Kalibrationsparameter angibt und $\varepsilon$ eine Extinktionseigenschaft des zu messenden Gasgemischs angibt, die von den Konzentrationen der Absorbergase und den Wirkungsquerschnitten der Absorbergase abhängt, wobei die Bestimmung der theoretischen Funktion in dem zweiten Schritt durch Vorgabe von Zahlenwerten für die Wirkungsquerschnitte und den Geräteparameter und das Einsetzen von den Konzentrationen als anzupassende Parameter erfolgt. Bei dieser Ausführungsform wird somit die theoretische Funktion auf Basis des bekannten Beer-Lambert-Gesetz beschrieben, was anerkanntermaßen zu einer physikalisch exakten Definition der theoretischen Funktion führt. Dabei wird bei der Formulierung der theoretischen Funktion angenommen, dass $I(\lambda)$ die physikalisch zu erwartende Lichtintensität angibt, die als Messwerte während der Messung aus dem Detektor ausgelesen werden. $I_0(\lambda)$ gibt hingegen eine Ausgangslichtintensität an. Diese Ausgangslichtintensität kann beispielsweise bei einer Kalibrationsmessung ermittelt werden, in der eine vordefinierte Nullluft in der Messzelle angeordnet ist. Bei der Formulierung der theoretischen Funktion gibt $L$ den Kalibrationsparameter an, der den Zustand der Messzelle während der Messung berücksichtigt, wohingegen $\varepsilon$ den Funktionsteil der theoretischen Funktion beinhaltet, der die Absorptionseigenschaft des Gasgemischs berücksichtigt. $\varepsilon$ hängt sowohl von den Konzentrationen als auch von den Wirkungsquerschnitten der Absorbergase ab. $\varepsilon$ kann sowohl schmalbandige als auch breitbandige Extinktionen berücksichtigen, beispielsweise Absorptions- und/oder Streueffekte. Beispielsweise kann $\varepsilon$ nicht nur von den Konzentrationen der Absorbergase sondern auch von weiteren Extinktionsparametern abhängen. Die Wirkungsquerschnitte, von denen $\varepsilon$ abhängt, sind literaturbekannt und können somit als Zahlenwerte während des zweiten Schritts zur Bestimmung der theoretischen Funktion eingesetzt werden, so dass beispielsweise als wählbare Parameter $\varepsilon$ ausschließlich die Konzentrationen der Absorbergase und insbesondere als weitere Extinktionsparameter die Konzentrationen von Streugasen enthält.

In einer Ausführungsform wird als Kalibrationsparameter eine effektive Geräteweglänge $L_{eff}$ verwendet, wobei als Geräteparameter eine Weglänge $L_0$ verwendet wird, wobei $L_{eff}$ dargestellt wird als Produkt $L_{eff}(\lambda) = L_0(\lambda)*K(\lambda)$, wobei

$$K(\lambda) = \frac{D_{CE}(\lambda)}{\exp(D_{CE}(\lambda)) - 1} \,,$$

insbesondere $K(\lambda)$ dargestellt wird als wobei $D_{CE}$ dargestellt wird als

$$D_{CE}(\lambda) = \ln\left(1 + L_0(\lambda)\left(\sum_{i=1}^{G} x_i \cdot \sigma_i(\lambda) + f(\vec{m}, \lambda)\right)\right) ,$$

wobei $x_i$ die festgelegten Annahmewerte der Konzentrationen der Absorbergase und $\sigma_i$ vorbekannte Wirkungsquerschnitte der Absorbergase angeben, wobei von G verschiedenen Absorbergasen ausgegangen wird, wobei $f(\vec{m}, \lambda)$ eine breitbandige Abschwächung des Lichts in der Messzelle beschreibt, wobei $\vec{m}$ eine Menge an Extinktionsparametern $m_n$ angibt. Es versteht sich von selbst, dass das Verfahren für eine wählbare Anzahl G an unterschiedlichen Absorbergasen, insbesondere in einer besonders einfachen Ausführungsform auch für nur ein Absorbergas (G=1) durchgeführt werden kann. Die beschriebene Ausführungsform ermöglicht gleichzeitig eine physikalisch präzise Formulierung der theoretischen Funktion und die Durchführung einer Ausgleichsrechnung während des dritten Schritts eines jeden Zyklus, da die Parametrisierung der theoretischen Funktion über die während der Ausgleichsrechnung anzupassenden Parameter $x_i$ und $\vec{m}$ noch hinreichend einfach ist, um eine numerische Anpassung dieser wählbaren Parameter während der Ausgleichsrechnung zu ermöglichen. Der Kalibrationsparameter ist in Abhängigkeit von dem Geräteparameter $L_0$, den Konzentrationen $x_i$ und Wirkungsquerschnitten $\sigma_i(\lambda)$ der Absorbergase und in Abhängigkeit von Extinktionsparametern (über die Funktion $f(\vec{m}, \lambda)$) definiert. Sowohl für die Konzentrationen $x_i$ als auch für die Extinktionsparameter $m_n$ der Menge an Extinktionsparametern $\vec{m}$ werden vor dem ersten Schritt Annahmewerte festgelegt, über die während des zweiten Schritts die theoretische Funktion bestimmt wird. Im dritten Schritt werden sowohl die Extinktionsparameter $m_n$ der Menge an Extinktionsparametern $\vec{m}$ als auch die Konzentrationen $x_i$ der Absorbergase bei der Durchführung der Ausgleichsrechnung variiert. Die Funktion $f(\vec{m}, \lambda)$ wird in einer Ausführungsform des erfindungsgemäßen Verfahrens definiert über $f(\vec{m}, \lambda) = \varepsilon_{Mie}(\vec{m}_1, \lambda) + \Delta\varepsilon_{Raleigh}(\vec{m}_2, \lambda)$. Dem liegt die physikalische Überlegung zugrunde, dass die breitbandige Abschwächung des Lichts in der Messzelle im Wesentlichen auf Mie-Streuung und auf Rayleigh-Streuung an entsprechenden Streuern beruht. Der Extinktionsparametervektor $\vec{m}$, der die Menge an Extinktionsparametern beschreibt, umfasst die Extinktionsparameter $m_n$, die in den beiden verschiedenen Extinktionsparametervektoren $\vec{m}_1$ und $\vec{m}_2$ enthalten sind. In einer Ausführungsform wird die Funktion $f(\vec{m}, \lambda)$ als ein Polynom niedriger Ordnung, insbesondere ein Polynom $\leq$ 5. Ordnung, insbesondere ein Polynom $\leq$

$$f(\vec{m}, \lambda) = \sum_{n=0}^{N} m_n * \lambda^n \,.$$

3. Ordnung in der theoretischen Funktion definiert, d. h. In einer Ausführungsform wird die Funktion $f(\vec{m}, \lambda)$ angenähert durch eine Potenzfunktion $f(\vec{m}, \lambda) = m_1 * \lambda^{m_2}$, wobei die Extinktionsparameter $m_1$ und $m_2$ die während der Ausgleichsrechnung anzupassenden Parameter darstellen. Bei der Darstellung von $f(\vec{m}, \lambda)$ als Potenzfunktion ist eine besonders einfache Durchführung der Ausgleichsrechnung ermöglicht, da lediglich zwei Extinktionsparameter $m_1$, $m_2$ anzupassen sind. Eine solche Näherung für die Funktion $f(\vec{m}, \lambda)$ kann insbesondere dann physikalisch sinnvoll sein und zu einer sehr präzisen Ermittlung der Konzentrationen mit dem erfindungsgemäßen Verfahren führen, wenn sichergestellt ist, dass das zu messende Gasgemisch im Vergleich zu der bei einer Ausgangsintensitätsmessung zur Bestimmung von $I_0(\lambda)$ in der Messzelle vorgesehenen Nullluft keine abweichenden Rayleigh-Streuer sondern ausschließlich Mie-Streuer enthält.

In einer Ausführungsform wird während des zweiten Schritts in einem ersten Zwischenschritt für jedes Absorbergas ein spezifischer wellenlängenabhängiger theoretischer Absorptionsverlauf aus dem berechneten Kalibrationsparameter und einem vorbekannten Wirkungsquerschnitt des jeweiligen Absorbergases berechnet. In einem zweiten Zwischenschritt wird während des zweiten Schritts für jedes Absorbergas ein theoretisches Absorberspektrum durch mathematische Faltung des jeweiligen Absorptionsverlaufs mit einer vorgegebenen Instrumentenfunktion berechnet. Die theoretische Funktion wird definiert als Funktion von den Absorberspektren der jeweiligen Absorbergase als wellenlängenabhängige Zahlenwerte und von den Konzentrationen der Absorbergase als Parameter. Bei dieser Ausführungsform wird die theoretische Funktion physikalisch besonders exakt definiert, da der jeweilige Absorptionsverlauf des jeweiligen Absorbergases, in dem der wellenlängenabhängige Kalibrationsparameter des Messgeräts und der wellenlängenabhängige Wirkungsquerschnitt des jeweiligen Absorbergases enthalten sind, mathematisch mit der Instrumentenfunktion gefaltet wird zur Definition eines theoretischen Absorberspektrums, das dann für jedes Absorbergas in Abhängigkeit von der Konzentration des jeweiligen Absorbergases das theoretisch erwartete, mit dem Messgerät messbare Absorberspektrum angibt. Dabei ist zu berücksichtigen, dass sowohl der Kalibrationsparameter als auch der Wirkungsquerschnitt sehr stark von der Wellenlänge abhängen, so dass die mathematische Faltung des daraus ermittelten Absorptionsverlaufs mit der Instrumentenfunktion zur physikalisch möglichst exakten Bestimmung der theoretischen Funktion von erheblicher Bedeutung ist. Dadurch, dass für den Kalibrationsparameter durch die erfindungsgemäße, iterative Durchführung von Zyklen stets ein Wert eingesetzt wird, der unabhängig von den Konzentrationen als anzupassende Parameter ist, kann trotz der mathematischen Faltung bei dem erfindungsgemäßen Verfahren gewährleistet werden, dass die theoretische Funktion, über die im dritten Schritt eines jeden Zyklus die Ausgleichsrechnung durchgeführt wird, noch so einfach formuliert ist (in Abhängigkeit von den Konzentrationen und insbesondere weiteren Extinktionsparametern), dass die Ausgleichsrechnung numerisch durchführbar ist. Allgemein sei an dieser Stelle angemerkt, dass die Instrumentenfunktion eine Funktion ist, die die Charakteristik des Messverhaltens des spektroskopischen Messgeräts, insbesondere des Detektors berücksichtigt. So wird hierdurch beispielsweise die Sensitivität des Detektors und die Auflösung des Detektors bezogen auf die Wellenlänge berücksichtigt.

In einer besonders bevorzugten Ausführungsform wird der Kalibrationsparameter berechnet über

$$L_{eff}(\lambda) = L_0(\lambda) * K(\lambda) = L_0(\lambda) * \frac{D_{CE}(\lambda)}{\exp(D_{CE}(\lambda)) - 1} ,$$

wobei

$$D_{CE}(\lambda) = \ln\left[1 + L_0(\lambda)\left(\sum_{i=1}^{G} x_i \sigma_i(\lambda) + f(\vec{m}, \lambda)\right)\right],$$

wobei $L_{eff}(\lambda)$ den Kalibrationsparameter darstellt, $L_0(\lambda)$ den Geräteparameter darstellt, $\sigma_i$ den Wirkungsquerschnitt eines bestimmten Absorbergases und $x_i$ den festgelegten Annahmewert des bestimmten Absorbergases angibt, wobei von G verschiedenen Absorbergasen ausgegangen wird, $f(\vec{m}, \lambda)$ eine breitbandige Abschwächung von Licht in der Messzelle und $\vec{m}$ eine Menge an Extinktionsparametern angibt. Diese Ausführungsform beschreibt eine mathematisch möglichst einfache und gleichzeitig möglichst präzise Formulierung des Kalibrationsparameters und somit Bestimmung der theoretischen Funktion, wodurch die Ausgleichsrechnung in dem dritten Schritt eines jeden Zyklus besonders fehlerreduzierend durchgeführt werden kann, wodurch die Konzentrationen der Absorbergase in dem Gasgemisch möglichst exakt bestimmt werden können. Es versteht sich von selbst, dass das Verfahren für eine wählbare Anzahl G an unterschiedlichen Absorbergasen, insbesondere in einer besonders einfachen Ausführungsform auch für nur ein Absorbergas (G=1) durchgeführt werden kann. Die Erfinder haben erkannt, dass die Verwendung von spektral hoch aufgelösten $\sigma_i(\lambda)$ für eine genaue Bestimmung von $L_{eff}(\lambda)$ besonders vorteilhaft ist, da dies insbesondere dazu beitragen kann, dass die richtige Bestimmung von $L_{eff}(\lambda)$ nicht durch die Auflösung des Detektors limitiert ist und auch schmalbandige Absorptionsstrukturen, deren Halbwertsbreite kleiner oder gleich der Auflösung des Detektors ist, korrekt berücksichtigt werden können.

Besonders bevorzugt wird die mathematische Faltung zur Bestimmung des Absorberspektrums des bestimmten Absorbergases durchgeführt auf Basis der Gleichung

$$\Theta_i(\lambda) = \frac{1}{x_i} \ln\left[H(\lambda) \otimes e^{-L_{eff}*\sigma_i(\lambda)*x_i}\right],$$

wobei $\Theta_i(\lambda)$ das Absorberspektrum des bestimmten Absorbergases und $H(\lambda)$ die Instrumentenfunktion angibt, wobei insbesondere als Näherung dieser Gleichung die Faltung durchgeführt wird mit der Näherungsgleichung $\Theta_i(\lambda) = H(\lambda) \otimes (L_{eff}(\lambda) * \sigma_i(\lambda))$. Dabei ist $H(\lambda)$ die vorbekannte Instrumentenfunktion des optischen Messgeräts, insbesondere unter Berücksichtigung der Eigenschaften des Detektors des Messgeräts. Über die beschriebene Faltung können die Absorberspektren $\Theta_i(\lambda)$ physikalisch exakt bestimmt werden, wobei die Absorberspektren anschließend in dem zweiten Schritt eines jeden Zyklus zur Bestimmung der theoretischen Funktion verwendet werden können. Die Absorberspektren $\Theta_i(\lambda)$ können insbesondere dann besonders exakt durch die Näherung $\Theta_i(\lambda) = H(\lambda) \otimes (L_{eff}(\lambda) * \sigma i(\lambda))$ angenähert werden, wenn davon ausgegangen werden kann, dass das Gasgemisch keine stark absorbierenden Absorbergase enthält, die Absorptionsstrukturen aufweisen, die bezogen auf ihre Breite in Abhängigkeit von der Wellenlänge viel schmaler sind als die Auflösung des Detektors des Messgeräts bezogen auf die Wellenlänge. Die Erfinder haben erkannt, dass über die genannte Näherung, die bei einer Vielzahl an Gasgemischen angewandt werden kann, die Durchführung der Ausgleichsgleichung noch weiter vereinfacht werden kann, was ein schnelleres und kostengünstigeres Ermitteln der Konzentrationen über das erfindungsgemäße Verfahren ermöglicht.

In einer Ausführungsform wird die theoretische Funktion T aus den Absorberspektren in dem zweiten Schritt eines jeden Zyklus wie folgt berechnet: Es wird angenommen, dass die theoretische Funktion als Funktion $T$ definiert werden kann, die von den Konzentrationen $\vec{x}$, den Extinktionsparametern $\vec{m}$ und Polynomparametern $\vec{v}$ abhängt. Entsprechend wird in diesem Ausführungsbeispiel die theoretische Funktion definiert als

$$T = T(\vec{x}, \vec{m}, \vec{v}, \lambda) = \sum_{i=1}^{G} \Theta_i(\lambda) \cdot x_i + H(\lambda) \otimes \left[L_{eff}(\lambda) \cdot f(\vec{m}, \lambda)\right] + P(\vec{v}, \lambda) .$$

Dabei wird über $\vec{x}$ die Menge an verschiedenen Konzentrationen $x_i$, die jeweils einem der verschiedenen Absorbergase zugeordnet sind, dargestellt. Bei dem vorliegenden Fall wird von $G$ verschiedenen Absorbergasen ausgegangen, so dass $\vec{x}$ $G$ unterschiedliche $x_i$ enthält. $\vec{m}$ beschreibt die Menge an Extinktionsparametern und $\vec{v}$ beschreibt eine Menge an Polynomparametern. Bei einer bevorzugten Ausführungsform der genannten Ausführungsform wird der Summand der theoretischen Funktion $H(\lambda) \otimes \lfloor L_{eff}(\lambda) * f(\vec{m}, \lambda) \rfloor$ angenähert durch: $H(\lambda) \otimes \lfloor L_{eff}(\lambda) * f(\vec{m}, \lambda) \rfloor = L_{eff}(\lambda) * f(\vec{m}, \lambda)$. Durch diese Näherung kann die theoretische Funktion insbesondere weiterhin sehr exakt die Messwertfunktion physikalisch-mathematisch widerspiegeln, da die Wellenlängenabhängigkeit von $L_{eff}(\lambda) * f(\vec{m}, \lambda)$ relativ gering ist im Hinblick auf die Auflösung des Detektors, die in der Instrumentenfunktion $H(\lambda)$ enthalten ist. $P(\vec{v}, \lambda)$ beschreibt einen Breitbandparameter, über den eine breitbandige Extinktionseigenschaft des Gasgemischs in der Messzelle berücksichtigt wird. Bei der beschriebenen Ausführungsform wird der Breitbandparameter als von $\vec{x}$ und $\vec{m}$ unabhängiger, anzupassender Parameter während der Ausgleichsrechnung

$$P(\vec{v}, \lambda) = \sum_{i=0}^{N} v_i \lambda^i \ .$$

mit angepasst. Dabei kann $P(\vec{v}, \lambda)$ beispielsweise definiert werden als $P$ ist somit als Polynom definiert, wobei vorzugsweise $N \leq 5$, insbesondere $N = 3$ für die Definition der theoretischen Funktion gesetzt wird, da durch ein entsprechendes Polynom eine ausreichend gute Annäherung an ein breitbandiges Absorptionsspektrum erreicht werden kann und gleichzeitig die Anzahl der während der Ausgleichsrechnung anzupassenden Parameter möglichst geringgehalten werden kann. Wie oben erläutert kann auch $f(\vec{m}, \lambda)$ beispielsweise als Polynom J-ter Ordnung, d.

$$f(\vec{m}, \lambda) = \sum_{n=0}^{J} m_n \lambda^n \ ,$$

h. dargestellt werden , beispielsweise als Polynom mit $J \leq 5$, insbesondere als Polynom mit $J = 3$, also als Polynom 3. Ordnung, dargestellt werden, so dass dann vier Extinktionsparameter ($m_0, m_1, m_2, m_3$) in dem die Menge an Extinktionsparametern $\vec{m}$ definierenden Vektor enthalten sind. Dem Fachmann ist ersichtlich, dass bei dem beschriebenen Ausführungsbeispiel eine theoretische Funktion T definiert wird, in der sämtliche anzupassende Parameter linear enthalten sind. Diese Formulierung einer theoretischen Funktion, die ausschließlich linear von den während der Ausgleichsrechnung anzupassenden Parametern abhängt, ist allgemein besonders vorteilhaft, da dies eine möglichst einfache Realisierung der Ausgleichsrechnung ermöglicht und somit eine fehlerminimierende Annäherung der theoretischen Funktion an die Messwertfunktion. An diesem Ausführungsbeispiel wird ersichtlich, dass das erfindungsgemäße Verfahren mit der iterativen Durchführung von Zyklen überhaupt erst die Durchführung einer möglichst fehlerminimierenden Anpassungsrechnung auf Basis von physikalisch sehr exakt formulierten theoretischen Funktionen ermöglicht. Denn durch das iterative Verfahren wird gewährleistet, dass die anzupassenden Parameter, die in dem Kalibrationsparameter (in dem beschriebenen Ausführungsbeispiel $L_{eff}(\lambda)$) enthalten sind, bei der Definition der theoretischen Funktion nicht in komplexen funktionalen Abhängigkeiten in der theoretischen Funktion auftauchen, was die Durchführung einer Ausgleichsrechnung praktisch unmöglich machen würde. Wie in dem Ausführungsbeispiel gezeigt ermöglicht vielmehr das iterative Verfahren, dass selbst bei dem Durchführen einer Faltung zum Bestimmen der theoretischen Funktion hierdurch keine komplexen Abhängigkeiten der theoretischen Funktion von den anzupassenden Parametern entstehen. Dies ist dadurch bedingt, dass die Funktionen, die mit der Instrumentenfunktion gefaltet werden müssen, einfach gehalten werden können, da die anzupassenden Parameter in dem Kalibrationsparameter während der Faltung nur als Zahlenwerte enthalten sind.

In einer Ausführungsform wird festgelegt, wie oft der Zyklus hintereinander durchgeführt wird, wobei dies erfolgt, indem a) eine maximale Anzahl an Zyklen vorgegeben wird und festgelegt wird, dass bei Erreichen der maximalen Anzahl an Zyklen kein weiterer Zyklus durchgeführt wird, b) festgelegt wird, dass kein weiterer Zyklus durchgeführt wird, sobald die in dem zuletzt durchgeführten Zyklus ermittelten Annahmewerte für sämtliche Konzentrationen sich um weniger als einen Grenzwert von den Annahmewerten unterscheiden, die in dem vorangehenden Zyklus ermittelt wurden, und/oder c) festgelegt wird, dass kein weiterer Zyklus durchgeführt wird, sobald ein Residuum zwischen der Messwertfunktion und der in dem zuletzt durchgeführten Zyklus durch Festlegung der Annahmewerte ermittelten theoretischen Funktion geringer ist als ein vorgegebener Schwellwert. In einigen Ausführungsformen kann nur eine der drei genannten Alternativen der Festlegung erfolgen, in anderen Ausführungsformen erfolgt eine Kombination von zwei oder sämtlichen dreien der genannten Alternativen. Dabei kann festgelegt sein, dass kein weiterer Zyklus durchgeführt wird, sobald eine der in den drei Alternativen genannten Bedingungen erfüllt ist. Betreffend Alternative a) wird eine absolute maximale Anzahl an Zyklen vorgegeben, nach denen kein weiterer Zyklus durchgeführt wird. Betreffend Alternative b) werden die Annahmewerte, die in dem dritten Schritt eines bestimmten Zyklus ermittelt werden, gespeichert und mit den Annahmewerten verglichen, die im dritten Schritt des darauffolgenden Zyklus ermittelt werden. Insbesondere kann die Alternative b) so festgelegt werden, dass nur dann kein weiterer Zyklus durchgeführt wird, wenn die in dem zuletzt durchgeführten Zyklus ermittelten Annahmewerte für sämtliche während der Ausgleichsrechnung des dritten Schritts anzupassende Parameter der theoretischen Funktion, d. h. sämtliche Konzentrationen und insbesondere weitere anzupassende Parameter wie Extinktionsparameter und insbesondere Polynomparameter, sich um weniger als einen Grenzwert von den Annahmewerten unterscheiden, die in dem vorangehenden Zyklus für die jeweiligen Parameter ermittelt wurden.

Als Grenzwert kann beispielsweise ein fester Wert für einen jeden anzupassenden Parameter, insbesondere ein gemeinsamer Grenzwert für sämtliche Konzentrationen festgelegt sein. Beispielsweise kann als Grenzwert auch eine prozentuale Abweichung zwischen dem Annahmewert des jeweiligen Parameters in dem zuletzt durchgeführten Zyklus und dem Annahmewert des jeweiligen Parameters in dem vorangehenden Zyklus festgelegt sein. Beispielsweise kann der Grenzwert auch in Abhängigkeit von einem Fehlerwert des Fits, der während der Ausgleichsrechnung bestimmt wird und die Güte der Übereinstimmung der in der Ausgleichsrechnung ermittelten theoretischen Funktion mit der Messwertfunktion beschreibt, festgelegt sein. Betreffend die Alternative c) kann festgelegt sein, dass für jeden Zyklus während des dritten Schritts ein Residuum zwischen der Messwertfunktion und der in dem dritten Schritt ermittelten theoretischen Funktion bestimmt wird, das den Grad der Übereinstimmung der theoretischen Funktion mit der Messwertfunktion beschreibt. Es wird dann kein weiterer Zyklus durchgeführt, wenn das Residuum unter einem vorgegebenen Schwellwert liegt.

Besonders bevorzugt werden die Annahmewerte der Konzentrationen vor der Durchführung eines ersten Zyklus jeweils auf einen bestimmten Zahlenwert festgelegt, wobei der jeweilige bestimmte Zahlenwert aus einem Speicher des Messgeräts ausgelesen wird oder durch einen Benutzer manuell eingegeben wird, wobei insbesondere als der jeweilige bestimmte Zahlenwert der in einer vorangegangenen Messung ermittelte gemessene Wert der jeweiligen Konzentration verwendet wird. Der bestimmte Zahlenwert kann beispielsweise willkürlich festgelegt sein, beispielsweise als identisch 0, um für jede Messung einen identischen Ausgangspunkt bereitzustellen, von dem aus die iterative Durchführung der Zyklen erfolgt. Beispielsweise kann der Zahlenwert durch einen Benutzer manuell eingegeben werden, wobei der Benutzer den bestimmten Zahlenwert entsprechend seiner Erwartung an die in dem Gasgemisch vermuteten Konzentrationen wählen kann. Dadurch kann die Dauer des Verfahrens zum Ermitteln der Konzentrationen verkürzt werden, da von einem realistischen ersten Annahmewert ausgegangen wird, wodurch die Anzahl der Iterationen reduziert werden kann, um zu einem korrekten Annahmewert für die Konzentrationen bzw. der Ausgabe von korrekten gemessenen Werten für die Konzentrationen zu gelangen. Beispielsweise kann der von dem Benutzer eingegebene bestimmte Zahlenwert oder der aus dem Speicher ausgelesene bestimmte Zahlenwert der Zahlenwert sein, der in einer vorangegangenen Messung als gemessener Wert der jeweiligen Konzentrationen ermittelt wurde. Auch hierdurch kann unter der Voraussetzung, dass die vorangegangene Messung unter vergleichbaren Bedingungen, d. h. zu einem vergleichbaren Gasgemisch durchgeführt wurde, die Anzahl der Iterationen und damit die Dauer des erfindungsgemäßen Verfahrens verkürzt werden, bis möglichst exakte gemessene Werte für die Konzentrationen ermittelt werden können.

In einer Ausführungsform wird das Gasgemisch durch einen Aerosolfilter gefiltert, bevor es in die Messzelle gelangt. Dadurch kann eine Extinktionseigenschaft des Gasgemischs in der Messzelle, die auf Mie-Streuung beruht, im Wesentlichen ausgeschlossen werden. Dadurch kann die Formulierung der theoretischen Funktion vereinfacht werden, was zu einer kostengünstigeren und schnelleren Durchführung des erfindungsgemäßen Verfahrens führen kann. Beispielsweise kann die oben angeführte Funktion zur Berücksichtigung breitbandiger Extinktionseigenschaften des Gasgemischs $f(\vec{m}, \lambda)$ in diesem Fall vereinfacht geschrieben werden als $f(\lambda)=\Delta\varepsilon_{\text{Rayleigh}}(\lambda)$, wobei $\Delta\varepsilon_{Rayleigh}(\lambda)$ als der weitere Extinktionsparameter über die aus der Literatur bekannten Rayleigh-Streuquerschnitte und die Dichte des Gasgemischs berechnet werden kann, wenn der Druck und die Temperatur in dem Gasgemisch bekannt sind. Dadurch kann die theoretische Funktion definiert werden, ohne dass weitere Extinktionsparameter als anzupassende Parameter in die theoretische Funktion eingeführt werden müssen. Dadurch wird die Anzahl der während der Ausgleichsrechnung anzupassenden Parameter verringert, was eine schnellere und präzisere Ermittlung der Konzentrationen der Absorbergase in dem Gasgemisch ermöglichen kann.

Die Erfindung betrifft ferner ein spektroskopisches Messgerät, das eine Lichtquelle, eine Messzelle mit einem optischen Resonator, einen Detektor und eine Recheneinheit umfasst. Die Lichtquelle ist zum Aussenden eines Lichtstrahls ausgebildet, der entlang eines Lichtwegs durch einen Eingang in eine Messzelle eintritt und durch einen Ausgang aus der Messzelle austritt. Der Detektor ist außerhalb der Messzelle an dem Ausgang der Messzelle angeordnet und zum Ausgeben von wellenlängenabhängigen Messwerten für eine Lichtintensität von auf ihn auftreffendem Licht ausgebildet. Die Recheneinheit ist dazu ausgebildet, Messwerte aus dem Detektor auszulesen und einen wellenlängenabhängigen Verlauf der Lichtintensität als wellenlängenabhänge Messwertfunktion darzustellen. Die Recheneinheit ist ferner dazu ausgebildet, Konzentrationen von Absorbergasen, die in einem Gasgemisch enthalten sind, das in der Messzelle angeordnet ist, zu ermitteln über eine Anpassungsrechnung zwischen einer theoretischen wellenlängenabhängigen Funktion und der wellenlängenabhängigen Messwertfunktion, wobei die theoretische Funktion einen wellenlängenabhängigen Kalibrationsparameter zur Berücksichtigung des Zustands des Messgeräts sowie die Konzentrationen als während der Anpassungsrechnung anzupassende Parameter enthält. Erfindungsgemäß ist die Recheneinheit dazu ausgebildet die Ermittlung der Konzentrationen durchzuführen unter Definition des Kalibrationsparameters als Funktion von einem in der Recheneinheit gespeicherten wellenlängenabhängigen Geräteparameter und einem wellenlängenabhängigen Korrekturfaktor, der als Funktion von den Konzentrationen definiert ist, wobei die Recheneinheit ausgebildet ist zum Durchführen eines Zyklus umfassend eine Abfolge von Schritten, wobei a) in einem ersten Schritt der Abfolge ein Zahlenwert für den Korrekturfaktor aus festgelegten Annahmewerten der Konzentrationen über die den Korrekturfaktor definierende Funktion berechnet wird, b) in einem zweiten Schritt der Abfolge die theoretische Funktion bestimmt wird, wobei der Kalibrationsparameter aus dem in dem ersten Schritt berechneten Zahlenwert für den Korrekturfaktor berechnet wird, c) in einem dritten Schritt der Abfolge durch eine Ausgleichsrechnung zwischen der in dem zweiten Schritt bestimmten theoretischen Funktion und der Messwertfunktion Werte für die Konzentrationen ermittelt werden und als neue Annahmewerte festgelegt werden, wobei die Recheneinheit ausgebildet ist, den Zyklus mehrfach hintereinander durchzuführen und die in dem dritten Schritt des letzten Zyklus ermittelten Annahmewerte als gemessene Werte der Konzentrationen auszugeben. Das erfindungsgemäße spektroskopische Messgerät ist kostengünstig herstellbar und ermöglicht gleichzeitig eine sehr präzise Ermittlung von Konzentrationen von Absorbergasen in einem Gasgemisch. Dies ist, wie aus der obigen Beschreibung hervorgeht, darin begründet, dass aufgrund der Ausgestaltung des spektroskopischen Messgeräts, insbesondere der Recheneinheit des Messgeräts, die Konzentrationen auf Basis von präzise formulierten physikalischen Gleichungen bestimmt werden können, so dass keine komplizierten Stabilisierungsmaßnahmen, wie beispielsweise eine Stabilisierung der Lichtintensität der Lichtquelle des Messgeräts, wie dies bei üblichen spektroskopischen Messgeräten erforderlich ist, bei denen stark angenäherte physikalische Gleichungen zur Ermittlung der Konzentrationen verwendet werden, die entsprechende Stabilisierungsmaßnahmen des spektroskopischen Messgeräts erfordern. Insbesondere kann das erfindungsgemäße spektroskopische Messgerät auch dann eine sehr fehlerarme Ermittlung der Konzentrationen ermöglichen, wenn die von der Lichtquelle ausgesandte Lichtintensität zwischen einer Kalibrationsmessung zum Kalibrieren des Messgeräts zum Ermöglichen einer Auswertung der Messwerte, insbesondere zur Bestimmung des Geräteparameters, und der eigentlichen Messung zur Ermittlung der Konzentrationen um mehr als 10 % variiert.

In einer Ausführungsform ist die Messzelle als von der Umgebung gasdicht abgeschlossene Messzelle ausgebildet, wobei die Messzelle einen Zugang zum Einlassen des Gasgemischs in die Messzelle aufweist, wobei an dem Zugang ein Aerosolfilter zum Herausfiltern von Aerosolen aus dem in die Messzelle gelangenden Gasgemisch angeordnet ist. Diese Ausführungsform ermöglicht es, eine Mie-Streuung in der Messzelle während der Messung zumindest weitestgehend, insbesondere vollständig zu verhindern. Dadurch können in der Recheneinheit Funktionen zum Ermitteln der Konzentrationen hinterlegt werden, die sowohl mathematisch weniger komplex als auch physikalisch sehr exakt formuliert sind, so dass mit dem spektroskopischen Messgerät die Ermittlung von Konzentrationen außergewöhnlich schnell und präzise erfolgen kann.

Das erfindungsgemäße spektroskopische Messgerät kann weitere Merkmale und Vorteile aufweisen, die aus der Beschreibung des erfindungsgemäßen Verfahrens und der verschiedenen Ausführungsformen des erfindungsgemäßen Verfahrens ersichtlich werden.

Die Erfindung wird nachfolgend unter Bezugnahme auf drei Figuren anhand von Ausführungsbeispielen näher erläutert.

Es zeigen:

Figur 1: in einer vereinfachten schematischen Prinzipdarstellung den Aufbau eines erfindungsgemäßen Messgeräts;

Figur 2: in einem Flussdiagramm den Ablauf einer Ausführungsform eines erfindungsgemäßen Verfahrens;

Figur 3: in einer Prinzipdarstellung die geringe Fehleranfälligkeit des erfindungsgemäßen Verfahrens im Vergleich zu herkömmlichen Verfahren.

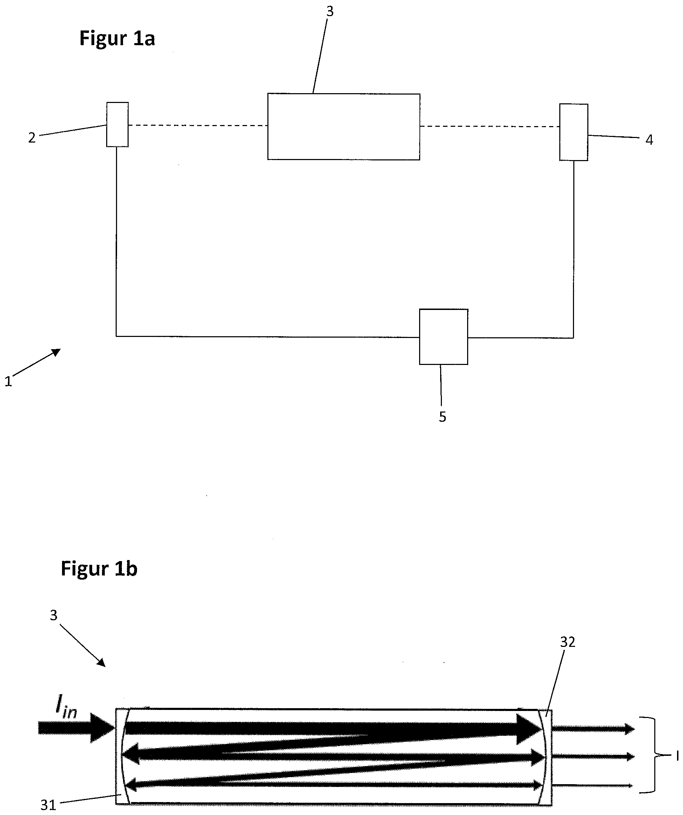

In Figur 1a ist der grundsätzliche Aufbau einer Ausführungsform eines erfindungsgemäßen spektroskopischen Messgeräts 1 dargestellt. Das spektroskopische Messgerät 1 umfasst eine Lichtquelle 2, die vorliegend als LED ausgebildet ist, sowie eine Messzelle 3, einen Detektor 4 und eine Recheneinheit 5. Bei dem spektroskopischen Messgerät 1 gemäß Figur 1a sind Lichtquelle 2, Recheneinheit 5 und Detektor 4 elektrisch miteinander verbunden, was in Figur 1 durch die durchgezogenen Linien dargestellt ist. Ferner ist in Figur 1 der Verlauf eines Lichtstrahls von der Lichtquelle 2 zum Detektor 4 rein schematisch über eine gestrichelte Linie dargestellt. Aus Figur 1a wird das grundlegende Prinzip spektroskopischer Messgeräte 1 deutlich: Eine Lichtquelle 2 emittiert einen Lichtstrahl, der durch einen Eingang in die Messzelle 3 des spektroskopischen Messgeräts 1 eintritt, in der Messzelle 3 einen optischen Lichtweg durchläuft und aus dem Ausgang aus der Messzelle 3 austritt, von wo er auf einen Detektor 4 gelangt. Mit dem Detektor 4 wird somit die Lichtintensität des Lichts gemessen, das von der Lichtquelle 2 ausgesandt wurde und durch die Messzelle 3 hindurchgetreten ist.

In Figur 1b wird ferner der grundsätzliche Aufbau einer Messzelle 3 schematisch dargestellt, die in einem spektroskopischen Messgerät 1 gemäß Figur 1a zum Einsatz kommt. Die Messzelle 3 weist eine Spiegelanordnung umfassend einen ersten Spiegel 31 und einen zweiten Spiegel 32 auf. Der erste Spiegel 31 stellt gleichzeitig den Eingang der Messzelle 3 dar, und der zweite Spiegel 32 stellt den Ausgang der Messzelle 3 dar. Licht, das über den ersten Spiegel 31 in die Messzelle 3 eingekoppelt wird, wird mehrfach zwischen erstem Spiegel 31 und zweitem Spiegel 32 hin und her reflektiert, wobei bei jedem Auftreffen von Licht auf den zweiten Spiegel 32 ein Teil des auftreffenden Lichts aus dem zweiten Spiegel 32 ausgekoppelt wird. Das aus dem zweiten Spiegel 32 ausgekoppelte Licht, d. h. aus dem Ausgang der Messzelle 3 austretende Licht, wird über herkömmliche optische Einrichtungen, wie beispielsweise Linsenanordnungen, zu einem Detektor 4 gelenkt, auf den das ausgekoppelte Licht, d. h. das aus dem Ausgang austretende Licht auftrifft, wobei der Detektor 4 als Messwert die Lichtintensität des auf ihn auftreffenden Lichts in Abhängigkeit von der Wellenlänge ausgibt. Die von dem Detektor 4 ausgegebenen Messwerte werden in der Recheneinheit 5 aufgezeichnet und ausgewertet zum Ermitteln der Konzentrationen von Absorbergasen in dem Gasgemisch, das in der Messzelle 3 des spektroskopischen Messgeräts 1 angeordnet ist. Insbesondere aus Figur 1b ist ersichtlich, dass die Weglänge, die das Licht in der Messzelle 3 zurücklegt, sowohl von der Reflektivität der Spiegelanordnung der Messzelle 3 als auch von den Extinktionseigenschaften des Gasgemischs in der Messzelle 3 abhängt. So verkürzt sich beispielsweise die mittlere Weglänge, die das Licht in der Messzelle 3 zurücklegt, bevor es an dem Ausgang aus der Messzelle 3 austritt, wenn in der Messzelle 3 ein Gasgemisch mit starken Absorptionseigenschaften angeordnet ist, da dann das Licht von dem Gasgemisch absorbiert wird, bevor es sehr häufig zwischen den Spiegel 31, 32 der Messzelle 3 hin und her reflektiert werden kann.

Bei dem erfindungsgemäßen Messgerät 1 ist die Recheneinheit 5 dazu ausgebildet, aus den Messwerten, die die Recheneinheit 5 aus dem Detektor 4 ausliest, eine wellenlängenabhängige Messwertfunktion darzustellen und Konzentrationen von Absorbergasen über eine Anpassungsrechnung zwischen einer theoretischen wellenlängenabhängigen Funktion und der wellenlängenabhängigen Messwertfunktion zu ermitteln. Dabei ist die Recheneinheit 5 so ausgebildet, dass sie die theoretische Funktion definiert als Funktion von einem wellenlängenabhängigen Kalibrationsparameter zur Berücksichtigung des Zustands des Messgeräts. Dabei ist die Recheneinheit 5 dazu ausgebildet, die Ermittlung der Konzentrationen durchzuführen unter Definition des Kalibrationsparameters als Funktion von einem in der Recheneinheit 5 gespeicherten wellenlängenabhängigen Geräteparameter und einem wellenlängenabhängigen Korrekturfaktor, der als Funktion von den Konzentrationen definiert ist, wobei die Recheneinheit ausgebildet ist zum Durchführen eines Zyklus umfassend eine Abfolge von Schritten, wobei a) in einem ersten Schritt der Abfolge ein Zahlenwert für den Korrekturfaktor aus festgelegten Annahmewerten der Konzentrationen über die den Korrekturfaktor definierende Funktion berechnet wird, b) in einem zweiten Schritt der Abfolge die theoretische Funktion bestimmt wird, wobei der Kalibrationsparameter aus dem in dem ersten Schritt berechneten Zahlenwert für den Korrekturfaktor berechnet wird, c) in einem dritten Schritt der Abfolge durch eine Ausgleichsrechnung zwischen der in dem zweiten Schritt bestimmten theoretischen Funktion und der Messwertfunktion Werte für die Konzentrationen ermittelt werden und als neue Annahmewerte festgelegt werden, wobei die Recheneinheit 5 ausgebildet ist, den Zyklus mehrfach hintereinander durchzuführen und die in dem dritten Schritt des letzten Zyklus ermittelten Annahmewerte als gemessene Werte der Konzentrationen auszugeben.

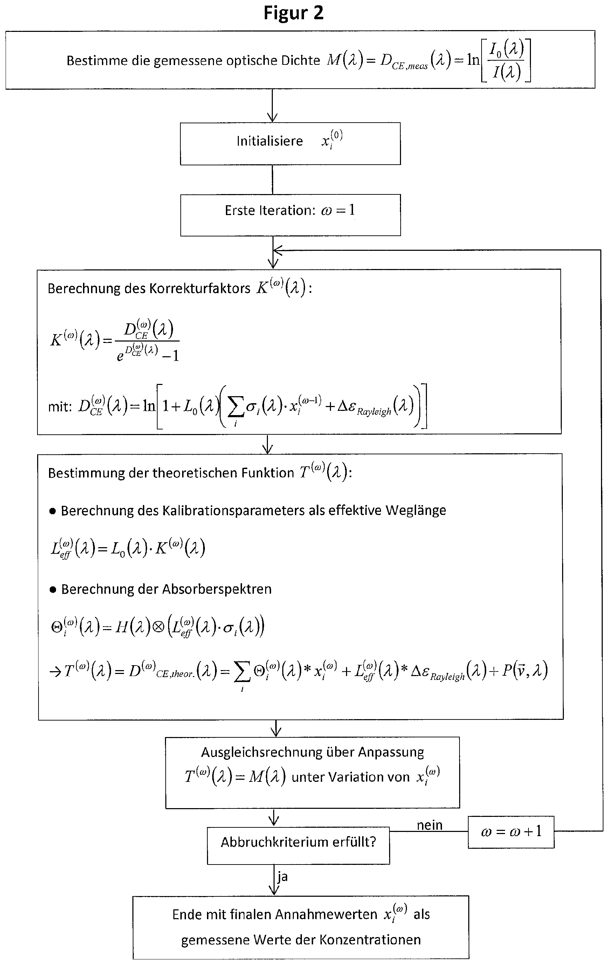

Die verschiedenen Schritte, die die Recheneinheit 5 des spektroskopischen Messgeräts 1 durchführt, und die den Schritten einer entsprechenden Ausführungsform eines erfindungsgemäßen Verfahrens bei der Ermittlung von Konzentrationen von Absorbergasen entsprechen, sind in Figur 2 schematisch in einem Flussdiagramm dargestellt. Zunächst ermittelt die Recheneinheit 5 aus den aus dem Detektor 4 ausgelesenen Messwerten eine wellenlängenabhängige Messwertfunktion $M(\lambda)$. In dem vorliegenden Ausführungsbeispiel wird als Messwertfunktion die optische Dichte

$$M(\lambda) = D_{CE,meas}(\lambda) = \ln\left[\frac{I_0(\lambda)}{I(\lambda)}\right]. \quad I(\lambda)$$

$D_{CE,meas}(\lambda)$ definiert über                                        ist dabei der wellenlängenabhängige Verlauf der Messwerte der Lichtintensität, die aus dem Detektor 4 während der Messung ausgelesen wurden, wohingegen $I_0(\lambda)$ der wellenlängenabhängige Verlauf der Messwerte während einer Ausgangsmessung ist, in der Nullluft in der Messzelle angeordnet ist, vorliegend Stickstoff ($N_2$). $I_0(\lambda)$ wurde während einer Ausgangsmessung ermittelt, die vor der Messung zur Ermittlung der Konzentrationen der Absorbergase in dem Gasgemisch durchgeführt wurde, und stellt somit eine Ausgangslichtintensität dar. $I_0(\lambda)$ ist in der Recheneinheit 5 abgespeichert. Anschließend werden die Annahmewerte für die Konzentrationen $x_i$ vor Durchführung eines ersten Zyklus jeweils auf einen Ausgangswert festgelegt, der in Figur 2 mit $x_i^{(0)}$ bezeichnet ist. Dieser Ausgangswert wird in dem beschriebenen Ausführungsbeispiel aus einem Speicher der Recheneinheit 5 ausgelesen, in dem der in der vorangegangenen Messung ermittelte Wert für die Konzentrationen $x_i$ hinterlegt ist. Dieser Wert aus der vorangegangenen Messung wird als Ausgangswert $x_i^{(0)}$ festgelegt. Sodann wird der Zyklus mehrfach hintereinander durchgeführt. Bei jedem Zyklus wird zunächst der festgelegte Annahmewert (in dem ersten Zyklus $x_i^{(0)}$) als Zahlenwert für die jeweiligen Konzentrationen eingesetzt, woraus über entsprechende mathematische Gleichungen der Korrekturfaktor $K^{(\omega)}(\lambda)$ und hierüber der Kalibrationsparameter $L_{eff}^{(\omega)}(\lambda)$ für den Zyklus $\omega$ bestimmt werden. Dieser Kalibrationsparameter, der vorliegend als effektive Weglänge definiert ist, wird sodann zur Bestimmung der theoretischen Funktion verwendet. Bei dem beschriebenen Ausführungsbeispiel werden hierzu zunächst Absorberspektren $\Theta_i^{(\omega)}(\lambda)$ für jedes bestimmte Absorbergas $i$ ermittelt, wonach dann die theoretische Funktion

$$T^{(\omega)}(\lambda) = D^{(\omega)}_{CE,theor.}(\lambda) = \sum_i \Theta_i^{(\omega)}(\lambda) * x_i^{(\omega)} + L_{eff}^{(\omega)}(\lambda) * \Delta\varepsilon_{Rayleigh}(\lambda) + P(\vec{v},\lambda),$$

bestimmt wird über                                                                                                                     wobei

$$P(\vec{v},\lambda) = \sum_j v_j * \lambda^j .$$

Dabei ist zu berücksichtigen, dass bei der beschriebenen Ausführungsform das spektroskopische Messgerät 1 einen Zugang zum Einlassen des Gasgemischs in die Messzelle 3 aufweist, wobei an dem Zugang ein Aerosolfilter zum Herausfiltern von Aerosolen aus dem in die Messzelle 3 gelangenen Gasgemisch angeordnet ist, so dass die Mie-Streuung in der Messzelle 3 bei der Formulierung der theoretischen Funktion $T$ vernachlässigt werden kann. $\Delta\varepsilon_{Rayleigh}(\lambda)$ wird bei dem beschriebenen Ausführungsbeispiel mathematisch berechnet durch Messung von Temperatur und Druck über entsprechende Sensoren des spektroskopischen Messgeräts 1 und über Auslesen von Rayleigh-Streuquerschnitten aus einem Speicher der Recheneinheit 5. Die theoretische Funktion enthält bei dem beschriebenen Ausführungsbeispiel somit ausschließlich die Konzentrationen $x_i$ der Absorbergase als anzupassende Parameter. Anschließend wird in demselben Zyklus $\omega$ eine Ausgleichsrechnung zwischen Messwertfunktion $M(\lambda)$ und theoretischer Funktion $T^{(\omega)}(\lambda)$ durchgeführt, wobei die Konzentrationen $x_i^{(\omega)}$ verändert werden, damit die theoretische Funktion möglichst gut an die Messwertfunktion angenähert wird. Nach Durchführung der Ausgleichsrechnung wird sodann bestimmt, ob ein weiterer Zyklus durchgeführt wird. Hierzu wird ein Abbruchkriterium definiert und geprüft, ob das Abbruchkriterium erfüllt ist. Vorliegend besteht das Abbruchkriterium darin, dass kein weiterer Zyklus $\omega$+1 durchgeführt wird, wenn die in dem zuletzt durchgeführten Zyklus $\omega$ ermittelten Annahmewerte $x_i^{(\omega)}$ sich um weniger als 2 % von den Annahmewerten $x_i^{(\omega-1)}$ unterscheiden, die in dem vorangehenden Zyklus $\omega$-1 ermittelt wurden. Falls das Abbruchkriterium nicht erfüllt ist, wird ein weiterer Zyklus $\omega$+1 durchgeführt, wobei die Annahmewerte $x_i^{(\omega)}$ zur Bestimmung des Korrekturfaktors in diesem Zyklus $\omega$+1 eingesetzt werden. Sobald das Abbruchkriterium erfüllt ist, werden die in dem letzten Zyklus ermittelten Annahmewerte $x_i^{(\omega)}$ als gemessene Werte der Konzentrationen ausgegeben.

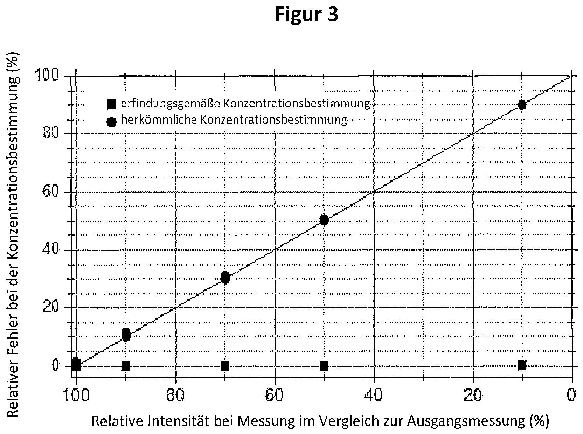

Aus Figur 3 wird ein wesentlicher Vorteil des erfindungsgemäßen Verfahrens bzw. des erfindungsgemäßen spektroskopischen Messgeräts 1 ersichtlich. In Figur 3 sind die relativen Fehler bei der Konzentrationsbestimmung in Abhängigkeit von einer relativen Intensitätsschwankung des von der Lichtquelle 2 des spektroskopischen Messgeräts 1 ausgesandten Lichts dargestellt, die zwischen einer Ausgangsmessung zum Ermitteln von $I_0(\lambda)$ und der Durchführung der eigentlichen Messung zum Ermitteln von $I(\lambda)$ bei dem beschriebenen Ausführungsbeispiel gemäß den Figuren 1 und 2 erfolgt. Diese relativen Fehler des erfindungsgemäßen spektroskopischen Messgeräts 1 werden zusammen mit relativen Fehlern eines herkömmlichen spektroskopischen Messgeräts 1 dargestellt, bei dem die Lichtquelle 2 entsprechende Schwankungen der Lichtintensität aufweist. Dabei ist in Figur 3 auf der X-Achse das Verhältnis zwischen der von der Lichtquelle ausgesandten Intensität während der Ausgangsmessung zur Bestimmung von $I_0(\lambda)$ und der von der Lichtquelle ausgesandten Lichtintensität während der eigentlichen Messung zur Bestimmung von $I(\lambda)$ angegeben. Aus Figur 3 ist klar zu entnehmen, dass mit Hilfe des erfindungsgemäßen spektroskopischen Messgeräts 1 selbst bei erheblichen Schwankungen der Lichtintensität, die die Lichtquelle 2 des spektroskopischen Messgeräts 1 aussendet, weiterhin eine Bestimmung der Konzentrationen von Absorbern in dem Gasgemisch in der Messzelle 3 mit einem sehr geringen Fehler durchgeführt werden kann. Bei herkömmlichen spektroskopischen Messgeräten ist dies jedoch nicht möglich, weshalb bei herkömmlichen spektroskopischen Messgeräten eine aufwendige, kostspielige und zumeist nicht vollkommen zufriedenstellende Stabilisierung der Lichtintensität, die die Lichtquelle 2 des Messgeräts 1 ausstrahlt, und Stabilisierung der Optik erforderlich ist.

Aus den beschriebenen Ausführungsbeispielen wird somit ersichtlich, dass das erfindungsgemäße spektroskopische Messgerät 1 bzw. das erfindungsgemäße Verfahren eine fehlerfreie Bestimmung von Konzentrationen von Absorbergasen in einem Gasgemisch ermöglicht, ohne dass aufwendige Maßnahmen zur Stabilisierung des Messgeräts 1 während der Durchführung von Messungen getroffen werden müssen.

**Bezugszeichenliste**

| | |
|---|---|
| 1 | spektroskopisches Messgerät |
| 2 | Lichtquelle |
| 3 | Messzelle |
| 4 | Detektor |
| 5 | Recheneinheit |
| 31 | erster Spiegel |
| 32 | zweiter Spiegel |

**Patentansprüche**

**1.** Verfahren zum Ermitteln von zumindest einer Konzentration von zumindest einem Absorbergas in einem zu messenden Gasgemisch mittels eines spektroskopischen Messgeräts (1), das eine Lichtquelle (2), eine Messzelle (3) mit einem optischen Resonator, einen Detektor (4) sowie eine Recheneinheit (5) umfasst, wobei das Gasgemisch in der Messzelle (3) angeordnet ist und mittels der Lichtquelle (2) ein Lichtstrahl durch einen Eingang der Messzelle (3) in die Messzelle (3) gesandt wird, wobei mittels des Detektors (4) wellenlängenabhängige Messwerte für eine Lichtintensität von aus einem Ausgang der Messzelle (3) austretendem Licht ermittelt werden, wobei ein wellenlängenabhängiger Verlauf der Lichtintensität als eine wellenlängenabhängige Messwertfunktion dargestellt wird und wobei eine Anpassungsrechnung zwischen einer theoretischen wellenlängenabhängigen Funktion und der wellenlängenabhängigen Messwertfunktion durchgeführt wird, wobei die theoretische Funktion einen wellenlängenabhängigen Kalibrationsparameter zur Berücksichtigung des Zustands des Messgeräts (1) sowie die zumindest eine Konzentration des zumindest einen in dem Gasgemisch enthaltenen Absorbergases als während der Anpassungsrechnung anzupassende Parameter enthält,
**dadurch gekennzeichnet, dass**
zur Durchführung der Anpassungsrechnung der Kalibrationsparameter als Funktion von einem vorbestimmten wellenlängenabhängigen Geräteparameter, der eine Weglänge eines Lichtwegs, den der Lichtstrahl in der Messzelle zurücklegt, charakterisiert, und einem wellenlängenabhängigen Korrekturfaktor definiert wird, wobei der Korrekturfaktor als Funktion von der zumindest einen Konzentration definiert wird, wobei ein Zyklus umfassend eine Abfolge von Schritten mehrfach hintereinander durchgeführt wird, wobei

a) in einem ersten Schritt der Abfolge ein Zahlenwert für den Korrekturfaktor aus einem festgelegten Annahmewert für die zumindest eine Konzentration über die den Korrekturfaktor definierende Funktion berechnet wird,
b) in einem zweiten Schritt der Abfolge die theoretische Funktion bestimmt wird, wobei der Kalibrationsparameter aus dem in dem ersten Schritt berechneten Zahlenwert für den Korrekturfaktor berechnet wird,
c) in einem dritten Schritt der Abfolge durch einen Abgleich zwischen der in dem zweiten Schritt bestimmten theoretischen Funktion und der Messwertfunktion eine Ausgleichsrechnung durchgeführt wird, wobei hierzu die zumindest eine in der theoretischen Funktion als frei wählbarer Parameter enthaltene Konzentration angepasst wird, um die in dem zweiten Schritt ermittelte theoretische Funktion möglichst nah an die Messwertfunktion anzupassen, und ein Wert für die zumindest eine Konzentration ermittelt wird und als neuer Annahmewert für die Konzentration festgelegt wird,

wobei der in dem dritten Schritt des letzten Zyklus ermittelte Annahmewert als gemessener Wert der zumindest einen Konzentration ausgegeben wird.

**2.** Verfahren nach Anspruch 1,
**dadurch gekennzeichnet, dass**
während der Ausgleichsrechnung die zumindest eine Konzentration als Parameter der theoretischen Funktion verändert wird zum Verringern der Unterschiede zwischen der theoretischen Funktion und der Messwertfunktion.

**3.** Verfahren nach einem der vorangehenden Ansprüche,
**dadurch gekennzeichnet, dass**
der Geräteparameter durch eine Kalibrationsmessung mit dem Messgerät (1) ermittelt wird, wobei die Ermittlung des gemessenen Wertes der zumindest einen Konzentration des zumindest einen Absorbergases durch die Anpassungsrechnung auf Basis von Messwerten erfolgt, die in einer Messung ermittelt werden, die getrennt von der Kalibrationsmessung durchgeführt wird.

**4.** Verfahren nach einem der vorangehenden Ansprüche,
**dadurch gekennzeichnet, dass**

die theoretische Funktion definiert wird als Summe aus einem ersten Summanden, der von dem Kalibrationsparameter und einem ausschließlich schmalbandige Absorptionseigenschaften des Gasgemischs beschreibenden Funktionsteil, der in Abhängigkeit von der zumindest einen Konzentration als anzupassender Parameter definiert ist, abhängt, und einem zweiten Summanden, der als von der zumindest einen Konzentration und dem Kalibrationsparameter unabhängiger Breitbandparameter definiert ist.

5. Verfahren nach einem der vorangehenden Ansprüche,
   **dadurch gekennzeichnet, dass**
   der Kalibrationsparameter so gewählt wird, dass er eine Weglänge eines Lichtwegs des Lichts in der Messzelle (3) charakterisiert, wobei die theoretische Funktion definiert wird auf Basis einer Darstellung der Lichtintensität $I(\lambda)$ als $I(\lambda)=I_0(\lambda)\cdot\exp(-L\cdot\varepsilon)$, wobei $I_0(\lambda)$ die Lichtintensität des austretenden Lichts bei Anordnung eines Gasgemischs ohne oder mit bekannter Extinktionseigenschaft in der Messzelle (3) angibt, $L$ den Kalibrationsparameter angibt und $\varepsilon$ eine Extinktionseigenschaft des zu messenden Gasgemischs angibt, die von der zumindest einen Konzentration des zumindest einen Absorbergases und dem zumindest einen Wirkungsquerschnitt des zumindest einen Absorbergases abhängt, wobei die Bestimmung der theoretischen Funktion in dem zweiten Schritt durch Vorgabe von Zahlenwerten für den zumindest einen Wirkungsquerschnitt und den Geräteparameter und das Einsetzen von der zumindest einen Konzentration als anzupassender Parameter erfolgt.

6. Verfahren nach einem der vorangehenden Ansprüche,
   **dadurch gekennzeichnet, dass**
   als Kalibrationsparameter eine effektive Weglänge $L_{eff}$ verwendet wird, wobei als Geräteparameter eine Geräteweglänge $L_0$ verwendet wird, wobei $L_{eff}$ dargestellt wird als Produkt $L_{eff}(\lambda)=L_0(\lambda)*K(\lambda)$, wobei insbesondere $K(\lambda)$

$$K(\lambda) = \frac{D_{CE}(\lambda)}{\exp(D_{CE}(\lambda))-1}$$

dargestellt wird als , wobei $D_{CE}$ dargestellt wird als

$$D_{CE}(\lambda) = \ln\left[1 + L_0(\lambda)\left(\sum_{i=1}^{G} x_i * \sigma_i(\lambda) + f(\vec{m},\lambda)\right)\right],$$

wobei $x_i$ die festgelegten Annahmewerte der Konzentrationen der Absorbergase und $\sigma_i$ vorbekannte Wirkungsquerschnitte der Absorbergase angeben, wobei von $G$ verschiedenen Absorbergasen ausgegangen wird, wobei $f(\vec{m},\lambda)$ eine breitbandige Abschwächung des Lichts in der Messzelle (3) beschreibt, wobei $\vec{m}$ eine Menge an Extinktionsparametern $m_n$ angibt.

7. Verfahren nach einem der vorangehenden Ansprüche,
   **dadurch gekennzeichnet, dass**
   während des zweiten Schritts in einem ersten Zwischenschritt für jedes Absorbergas ein spezifischer wellenlängenabhängiger theoretischer Absorptionsverlauf aus dem berechneten Kalibrationsparameter und einem vorbekannten Wirkungsquerschnitt des jeweiligen Absorbergases berechnet wird, wobei in einem zweiten Zwischenschritt für jedes Absorbergas ein theoretisches Absorberspektrum durch mathematische Faltung des jeweiligen Absorptionsverlaufs mit einer vorgegebenen Instrumentenfunkton berechnet wird, wobei die theoretische Funktion als Funktion von den Absorberspektren der jeweiligen Absorbergase als wellenlängenabhängige Zahlenwerte und von den Konzentrationen der Absorbergase als Parameter definiert wird.

8. Verfahren nach Anspruch 7,
   **dadurch gekennzeichnet, dass**

$$L_{eff}(\lambda) = L_0(\lambda) * K(\lambda) = L_0(\lambda) * \frac{D_{CE}(\lambda)}{\exp(D_{CE}(\lambda))-1}$$

der Kalibrationsparameter berechnet wird über , wobei

$$D_{CE}(\lambda) = \ln\left[1 + L_0(\lambda)\left(\sum_{i=1}^{G} x_i\sigma_i(\lambda) + f(\vec{m},\lambda)\right)\right]$$

, wobei $L_{eff}(\lambda)$ den Kalibrationsparameter darstellt, $L_0(\lambda)$ den Geräteparameter darstellt, $\sigma_i$ den Wirkungsquerschnitt eines bestimmten Absorbergases und $x_i$ den festgelegten Annahmewert des bestimmten Absorbergases angibt, wobei von $G$ verschiedenen Absorbergasen ausgegangen

wird, $f(\vec{m},\lambda)$ eine breitbandige Abschwächung von Licht in der Messzelle (3) und $\vec{m}$ eine Menge an Extinktionsparametern angibt.

9. Verfahren nach Anspruch 8,
**dadurch gekennzeichnet, dass**
die mathematische Faltung zur Bestimmung des Absorberspektrums des bestimmten Absorbergases durchgeführt

$$\Theta_i(\lambda) = \frac{1}{x_i}\ln\left[H(\lambda)\otimes e^{-L_{eff}\,^*\sigma_i(\lambda)^*x_i}\right]$$

wird auf Basis der Gleichung , wobei $\Theta_i(\lambda)$ das Absorberspektrum des bestimmten Absorbergases und $H(\lambda)$ die Instrumentenfunktion angibt, wobei insbesondere als Näherung dieser Gleichung die Faltung durchgeführt wird mit der Näherungsgleichung $\Theta_i(\lambda)=H(\lambda)\otimes L_{eff}(\lambda)^*\sigma_i(\lambda))$.

10. Verfahren nach einem der vorangehenden Ansprüche,
**dadurch gekennzeichnet, dass**
festgelegt wird, wie oft der Zyklus hintereinander durchgeführt wird, wobei dies erfolgt, indem

a) eine maximale Anzahl an Zyklen vorgegeben wird und festgelegt wird, dass bei Erreichen der maximalen Anzahl an Zyklen kein weiterer Zyklus durchgeführt wird,
b) festgelegt wird, dass kein weiterer Zyklus durchgeführt wird, sobald die in dem zuletzt durchgeführten Zyklus ermittelten Annahmewerte für sämtliche Konzentrationen sich um weniger als einen Grenzwert von den Annahmewerten unterscheiden, die in dem vorangehenden Zyklus ermittelt wurden, und/oder
c) festgelegt wird, dass kein weiterer Zyklus durchgeführt wird, sobald ein Residuum zwischen der Messwertfunktion und der in dem zuletzt durchgeführten Zyklus durch Festlegung der Annahmewerte ermittelten theoretischen Funktion geringer ist als ein vorgegebener Schwellwert.

11. Verfahren nach einem der vorangehenden Ansprüche,
**dadurch gekennzeichnet, dass**
der zumindest eine Annahmewert der zumindest einen Konzentration vor der Durchführung eines ersten Zyklus jeweils auf einen bestimmten Zahlenwert festgelegt wird, wobei der jeweilige bestimmte Zahlenwert aus einem Speicher des Messgeräts (1) ausgelesen wird oder durch einen Benutzer manuell eingegeben wird, wobei insbesondere als der jeweilige bestimmte Zahlenwert der in einer vorangegangenen Messung ermittelte gemessene Wert der jeweiligen Konzentration verwendet wird.

12. Verfahren nach einem der vorangehenden Ansprüche,
**dadurch gekennzeichnet, dass**
das Gasgemisch durch einen Aerosolfilter gefiltert wird, bevor es in die Messzelle (3) gelangt.

13. Spektroskopisches Messgerät (1) umfassend eine Lichtquelle (2), eine Messzelle (3) mit einem optischen Resonator, einen Detektor (4) sowie eine Recheneinheit (5), wobei die Lichtquelle (2) zum Aussenden eines Lichtstrahls ausgebildet ist, der entlang eines Lichtwegs durch einen Eingang in eine Messzelle (3) eintritt und durch einen Ausgang aus der Messzelle (3) austritt, wobei der Detektor (4) außerhalb der Messzelle (3) an dem Ausgang angeordnet ist und zum Ausgeben von wellenlängenabhängigen Messwerten für eine Lichtintensität von auf ihn auftreffendem Licht ausgebildet ist, wobei die Recheneinheit (5) ausgebildet ist zum Auslesen der Messwerte aus dem Detektor (4) und zum Darstellen eines wellenlängenabhängigen Verlaufs der Lichtintensität als wellenlängenabhängige Messwertfunktion sowie zum Ermitteln der Konzentration von zumindest einem Absorbergas über eine Anpassungsrechnung zwischen einer theoretischen wellenlängenabhängigen Funktion und der wellenlängenabhängigen Messwertfunktion, wobei die theoretische Funktion einen wellenlängenabhängigen Kalibrationsparameter zur Berücksichtigung des Zustands des Messgeräts (1) sowie die zumindest eine Konzentration des zumindest einen in dem Gasgemisch enthaltenen Absorbergases als während der Anpassungsrechnung anzupassende Parameter enthält,
**dadurch gekennzeichnet, dass**
die Recheneinheit (5) dazu ausgebildet ist, die Ermittlung der Konzentration von zumindest einem Absorbergas durchzuführen unter Definition des Kalibrationsparameters als Funktion von einem in der Recheneinheit (5) gespeicherten wellenlängenabhängigen Geräteparameter, der eine Weglänge eines Lichtwegs, den der Lichtstrahl in der Messzelle zurücklegt, charakterisiert, und einem wellenlängenabhängigen Korrekturfaktor, der als Funktion von der zumindest einen Konzentration definiert ist, wobei die Recheneinheit (5) ausgebildet ist zum Durchführen eines Zyklus umfassend eine Abfolge von Schritten, wobei

a) in einem ersten Schritt der Abfolge ein Zahlenwert für den Korrekturfaktor aus einem festgelegten Annahmewert für die zumindest eine Konzentration über die den Korrekturfaktor definierende Funktion berechnet wird,
b) in einem zweiten Schritt der Abfolge die theoretische Funktion bestimmt wird, wobei der Kalibrationsparameter aus dem in dem ersten Schritt berechneten Zahlenwert für den Korrekturfaktor berechnet wird,
c) in einem dritten Schritt der Abfolge durch einen Abgleich zwischen der in dem zweiten Schritt bestimmten theoretischen Funktion und der Messwertfunktion eine Ausgleichsrechnung durchgeführt wird, wobei hierzu die zumindest eine in der theoretischen Funktion als frei wählbarer Parameter enthaltene Konzentration angepasst wird, um die in dem zweiten Schritt ermittelte theoretische Funktion möglichst nah an die Messwertfunktion anzupassen, und ein Wert für die zumindest eine Konzentration ermittelt wird und als neuer Annahmewerte für die zumindest eine Konzentration festgelegt wird,

wobei die Recheneinheit (5) ausgebildet ist, den Zyklus mehrfach hintereinander durchzuführen und den zumindest einen in dem dritten Schritt des letzten Zyklus ermittelten Annahmewert als gemessenen Wert der zumindest einen Konzentration auszugeben.

**14.** Spektroskopisches Messgerät (1) nach Anspruch 13,
**dadurch gekennzeichnet, dass**
die Messzelle (3) als von der Umgebung gasdicht abgeschlossene Messzelle (3) ausgebildet ist, wobei die Messzelle (3) einen Zugang zum Einlassen des Gasgemischs in die Messzelle (3) aufweist, wobei an dem Zugang ein Aerosolfilter zum Herausfiltern von Aerosolen aus dem in die Messzelle (3) gelangenden Gasgemisch angeordnet ist.

## Claims

**1.** Method for determining at least one concentration of at least one absorbing gas in a gas mixture to be measured by means of a spectroscopic measuring device (1), which comprises a light source (2), a measuring cell (3) comprising an optical resonator, a detector (4) and a computer unit (5), wherein the gas mixture is arranged in the measuring cell (3) and a light beam is sent to the measuring cell (3) through an entrance of the measuring cell (3) by means of the light source (2), wherein wavelength-dependent measurement values for a light intensity of light leaving from an exit of the measuring cell are obtained by means of the detector (4), wherein a wavelength-dependent shape of the light intensity is represented as a wavelength-dependent measurement value function and wherein a curve fitting calculation between a theoretical wavelength-dependent function and the wavelength-dependent measurement value function is performed, wherein the theoretical function contains a wavelength-dependent calibration parameter for taking into account the state of the measuring device (1) as well as at least one concentration of the at least one absorbing gas contained in the gas mixture as a parameter to be fitted during the curve fitting calculation,
**characterized in, that**
for performing the curve fitting calculation, the calibration parameter is defined as a function of a predetermined wavelength-dependent device parameter characterizing a path length of the light which the light beam travels in the measuring cell and a wavelength-dependent correction factor, wherein the correction factor is defined as a function of at least one concentration, wherein a cycle comprising a sequence of steps is performed several times in a row, wherein

a) in a first step of the sequence, a numerical value for the correction factor is calculated from a stipulated assumed value for the at least one concentration using the function defining the correction factor,
b) in a second step of the sequence, the theoretical function is determined, wherein the calibration parameter is calculated from the numerical value for the correction factor calculated in the first step,
c) in a third step of the sequence, a value of the at least one concentration is obtained by a curve adjustment calculation between the theoretical function determined in the second step and the measurement value function and is stipulated as new assumed value for the concentration, wherein during the curve adjustment calculation the at least one concentration included in the theoretical function as freely selectable parameter is adjusted in order to adjust the theoretical function, which has been determined in the second step, as closely as possible to the measurement value function,

wherein the assumed value obtained in the third step of the last cycle is output as a measured value of the at least one concentration.

**2.** Method according to claim 1,
**characterized in that**
during the curve adjustment calculation, the at least one concentration as a parameter of the theoretical function is changed to reduce the differences between the theoretical function and the measurement value function.

**3.** Method according to one of the preceding claims,
**characterized in that**
the device parameter is obtained by a calibration measurement using the measuring device (1), wherein the measured value of the at least one concentration of the at least one absorbing gas is obtained by the curve fitting calculation which is based on measurement values obtained in a measurement performed separately from the calibration measurement.

**4.** Method according to one of the preceding claims,
**characterized in that**
the theoretical function is defined as a sum of a first summand, which depends on the calibration parameter and a function part that exclusively describes narrowband absorption properties of the gas mixture and is defined in dependence on the at least one concentration as a parameter to be adjusted, and a second summand that is defined as a broadband parameter being independent of the at least one concentration and the calibration parameter.

**5.** Method according to one of the preceding claims,
**characterized in that**
the calibration parameter is selected to characterize a path length of a light path of the light in the measuring cell (3), wherein the theoretical function is defined based on a representation of the light intensity $I(\lambda)$ as $I(\lambda)=I_0(\lambda) \cdot \exp(-L \cdot \varepsilon)$, where $I_0(\lambda)$ is the light intensity of the leaving light when a gas mixture without or with a known extinction property is arranged in the measuring cell (3), $L$ is the calibration parameter and $\varepsilon$ is an extinction property of the gas mixture to be measured, which depends on the at least one concentration of the at least one absorbing gas and the at least one cross section of the at least one absorbing gas, wherein the determination of the theoretical function in the second step is accomplished by predetermining numerical values for the at least one cross section and the device parameter and by using the at least one concentration as a parameter to be adjusted.

**6.** Method according to one of the preceding claims,
**characterized in that**
an effective path length $L_{eff}$ is used as a calibration parameter, wherein a device path length $L_0$ is used as a device parameter, wherein $L_{eff}$ is represented as a product $L_{eff}(\lambda)=L_0(\lambda)*K(\lambda)$, wherein in particular $K(\lambda)$ is represented as

$$K(\lambda) = \frac{D_{CE}(\lambda)}{\exp(D_{CE}(\lambda))-1},$$ wherein $D_{CE}$ is represented as

$$D_{CE}(\lambda) = \ln\left[1 + L_0(\lambda)\left(\sum_{i=1}^{G} x_i * \sigma_i(\lambda) + f(\vec{m},\lambda)\right)\right],$$ wherein $x_i$ represents the stipulated assumed values of the concentrations of the absorbing gases and $\sigma_i$ represents pre-known cross sections of the absorbing gases, wherein G different absorbing gases are assumed, wherein $f(\vec{m},\lambda)$ describes a broadband attenuation of the light in the measuring cell (3), wherein $\vec{m}$ is the quantity of extinction parameters $m_n$.

**7.** Method according to one of the preceding claims,
**characterized in that**
during a first intermediate step of the second step, a specific wavelength-dependent theoretical absorption shape is calculated from the calculated calibration parameter and a pre-known cross section of the respective absorbing gas wherein in a second intermediate step, a theoretical absorber spectrum is calculated for each absorbing gas by a convolution of the respective absorption shape with a predetermined instrument function, wherein the theoretical function is defined as function of the absorber spectra of the respective absorbing gases being a wavelength dependent number of numerical values and as function of the concentrations of the respective absorber gases as parameters.

**8.** Method according to claim 7,
**characterized in that**

$$L_{eff}(\lambda) = L_0(\lambda) * K(\lambda) = L_0(\lambda) * \frac{D_{CE}(\lambda)}{\exp(D_{CE}(\lambda)) - 1} \quad,$$

the calibration parameter is calculated using _____ wherein

$$D_{CE}(\lambda) = \ln\left[1 + L_0(\lambda)\left(\sum_{i=1}^{G} x_i \sigma_i(\lambda) + f(\vec{m}, \lambda)\right)\right],$$

where $L_{eff}(\lambda)$ is the calibration parameter, $I_0(\lambda)$ is the device parameter, $\sigma_i$ is the cross section of a particular absorbing gas and $x_i$ is the stipulated assumed value of the particular absorbing gas, wherein $G$ different absorbing gases are assumed, wherein $f(\vec{m}, \lambda)$ is a broadband attenuation of light in the measuring cell (3) and $\vec{m}$ is the quantity of extinction parameters.

**9.** Method according to claim 8,
**characterized in that**
the mathematical convolution for determining the absorber spectrum of the particular absorbing gas is performed

$$\Theta_i(\lambda) = \frac{1}{x_i}\ln\left[H(\lambda) \otimes e^{-L_{eff}*\sigma_i(\lambda)*x_i}\right],$$

based on the equation _____ where $\Theta_i(\lambda)$ represents the absorber spectrum of the particular absorbing gas and $H(\lambda)$ represents the instrument function, wherein in particular, as an approximation of this equation, the convolution is performed using the approximated equation $\Theta_i(\lambda) = H(\lambda) \otimes (L_{eff}(\lambda)*\sigma_i(\lambda))$.

**10.** Method according to one of the preceding claims,
**characterized in that**
the number of cycles which are performed in a row are defined according to

a) predefining a maximum number of cycles and defining that no further cycle is performed once the maximum number of cycles is reached,
b) stipulating that no further cycle is performed as soon as the assumed values obtained in the most recent cycle are different from the assumed values obtained in the previous cycle by less than a limit value for all concentrations
and/or
c) stipulating that no further cycle is performed as soon as a residuum between the measurement value function and the theoretical function determined in the most recent cycle by setting the assumed values is less than a predetermined threshold.

**11.** Method according to one of the preceding claims,
**characterized in that**
the at least one assumed value of the at least one concentration is respectively fixed to a particular numerical value prior to performing a first cycle, wherein the respective particular numerical value is read out from a memory of the measuring device (1) or is manually input by a user, wherein in particular the measured value of the respective concentration obtained in a previous measurement is used as the respective particular numerical value.

**12.** Method according to one of the preceding claims,
**characterized in that**
the gas mixture is filtered by an aerosol filter before reaching the measuring cell (3).

**13.** Spectroscopic measuring device (1), comprising a light source (2), a measuring cell (3) comprising an optical resonator, a detector (4) and a computing unit (5), wherein the light source is configured for emitting a light beam which enters through an entrance into the measuring cell (3) along a light path and emerges from the measuring cell (3) through an exit, wherein the detector (4) is arranged outside of the measuring cell (3) at the exit and configured to output a wavelength-dependent measurement value for a light intensity of light which hit thereon, wherein the computing unit (5) is configured to read out the measurement values from the detector (4) and to represent a wavelength-dependent shape of the light intensity as a wavelength-dependent measurement value function and also to determine the concentration of at least one absorbing gas using a curve fitting calculation between a theoretical wavelength-dependent function and the wavelength-dependent measurement value function, wherein the theoretical function includes a wavelength-dependent calibration parameter for taking into account the state of the measuring device (1) and at least one concentration of the at least one absorbing gas contained in the gas mixture as parameters to be adjusted during the curve fitting calculation,
**characterized in that**
the computing unit (5) is configured to perform the determination of the concentration of at least one absorbing gas under definition of the calibration parameter as a function of a wavelength-dependent device parameter characterizing a path length of the light which the light beam travels in the measuring cell and being stored in the computing unit (5) and a wavelength-dependent correction factor defined as a function of the at least one concentration, wherein the computing unit (5) is configured to perform a cycle comprising a sequence of steps, wherein

a) in a first step of the sequence, a numerical value for the correction factor is calculated from a stipulated assumed value for the at least one concentration using the function defining the correction factor,
b) in a second step of the sequence, the theoretical function is determined, wherein the calibration parameter is calculated from the numerical value for the correction factor calculated in the first step,
c) in a third step of the sequence, a value for the at least one concentration is obtained by a curve adjustment calculation between the theoretical function determined in the second step and the measurement value function and is used as a new assumed value for the at least one concentration, wherein during the curve adjustment calculation the at least one concentration included in the theoretical function as freely selectable parameter is adjusted in order to adjust the theoretical function, which has been determined in the second step, as closely as possible to the measurement value function,

wherein the computing unit (5) is configured to perform the cycle several times in a row and output the assumed value obtained in the third step of the last cycle as a measured value of the at least one concentration.

**14.** Spectroscopic measuring device (1) according to claim 13,
**characterized in that**
the measuring cell (3) is designed as a measuring cell (3) sealed in a gastight manner against the surroundings, wherein the measuring cell (3) has an entrance allowing the gas mixture to enter into the measuring cell (3), wherein an aerosol filter for filtering out aerosols from the gas mixture reaching the measuring cell (3) is disposed at the entrance.

**Revendications**

**1.** Procédé de détermination d'au moins une concentration d'au moins un gaz absorbant dans un mélange gazeux à mesurer au moyen d'un dispositif de mesure spectroscopique (1) qui comprend une source lumineuse (2), une cellule de mesure (3) avec un résonateur optique, un détecteur (4) et une unité informatique (5), le mélange gazeux étant disposé dans la cellule de mesure (3) et un faisceau lumineux étant transmis au moyen de la source lumineuse (2) à travers une entrée de la cellule de mesure (3) dans la cellule de mesure (3), des valeurs de mesure dépendant de la longueur d'onde étant déterminées au moyen du détecteur (4) pour une intensité lumineuse de lumière sortant d'une sortie de la cellule de mesure (3), dans lequel procédé un parcours dépendant de la longueur d'onde de l'intensité lumineuse est représenté sous la forme d'une fonction de valeur mesurée dépendant de la longueur d'onde et dans lequel un calcul d'adaptation est effectué entre une fonction théorique dépendant de la longueur d'onde et la fonction de valeur mesurée dépendant de la longueur d'onde, la fonction théorique contenant comme paramètre à adapter pendant le calcul d'adaptation un paramètre d'étalonnage dépendant de la longueur d'onde pour prendre en compte l'état du dispositif de mesure (1) et ladite au moins une concentration dudit au moins un gaz absorbant contenu dans le mélange gazeux,
**caractérise en ce que**,
pour effectuer le calcul d'adaptation, le paramètre d'étalonnage est défini en fonction d'un paramètre prédéterminé du dispositif dépendant de la longueur d'onde qui caractérise une longueur de trajet d'un trajet lumineux parcouru par le faisceau lumineux dans la cellule de mesure, et un facteur de correction dépendant de la longueur d'onde, le facteur de correction étant défini en fonction de ladite au moins une concentration, un cycle comprenant une séquence de pas étant effectué plusieurs fois de suite, dans laquelle

a) dans une première étape de la séquence, une valeur numérique pour le facteur de correction est calculée à partir d'une valeur supposée fixe pour ladite au moins une concentration via la fonction définissant le facteur de correction,
b) dans une deuxième étape de la séquence, la fonction théorique est déterminée, le paramètre d'étalonnage étant calculé à partir de la valeur numérique pour le facteur de correction calculée dans la première étape,
c) dans une troisième étape de la séquence, un calcul de compensation est effectué au moyen d'une comparaison entre la fonction théorique déterminée dans la deuxième étape et la fonction de la valeur mesurée, dans lequel calcul, à cette fin, ladite au moins une concentration contenue dans la fonction théorique est adaptée comme paramètre librement sélectionnable afin d'adapter la fonction théorique déterminée dans la deuxième étape aussi étroitement que possible à la fonction de la valeur mesurée et une valeur pour ladite au moins une concentration est déterminée et fixée comme nouvelle valeur supposée pour la concentration,

la valeur supposée déterminée dans la troisième étape du dernier cycle est délivrée sous la forme d'une valeur mesurée de ladite au moins une concentration.

2. Procédé selon la revendication 1,
**caractérisé en ce que**
pendant le calcul de compensation, ladite au moins une concentration est modifiée en tant que paramètre de la fonction théorique pour réduire les différences entre la fonction théorique et la fonction de la valeur mesurée.

3. Procédé selon l'une des revendications précédentes,
**caractérisé en ce que**
le paramètre de dispositif est déterminé par une mesure d'étalonnage avec le dispositif de mesure (1), la valeur mesurée de ladite au moins une concentration dudit au moins un gaz absorbant étant déterminée par le calcul d'adaptation sur la base de valeurs mesurées qui sont déterminées dans une mesure qui est effectuée séparément de la mesure d'étalonnage.

4. Procédé selon l'une des revendications précédentes,
**caractérisé en ce que**
la fonction théorique est définie comme la somme d'une première terme d'une somme qui dépend du paramètre d'étalonnage et d'une partie fonctionnelle décrivant exclusivement des propriétés d'absorption à bande étroite du mélange gazeux qui est définie comme un paramètre à ajuster en fonction de ladite au moins une concentration, et une seconde terme d'une somme qui est définie comme un paramètre à large bande indépendant de ladite au moins une concentration et le paramètre d'étalonnage.

5. Procédé selon l'une des revendications précédentes,
**caractérisé en ce que**
le paramètre d'étalonnage est sélectionné de telle sorte qu'il caractérise une longueur de trajet d'un trajet lumineux de la lumière dans la cellule de mesure (3), la fonction théorique étant définie sur la base d'une représentation de l'intensité lumineuse $I(\lambda)$ comme $I(\lambda)=I_0(\lambda)\cdot\exp(-L\cdot\varepsilon)$, $I_0(\lambda)$ indiquant l'intensité lumineuse de la lumière émergente quand un mélange gazeux est disposé dans la cellule de mesure (3) sans ou avec une propriété d'extinction connue, $L$ indiquant le paramètre d'étalonnage et $\varepsilon$ indiquant une propriété d'extinction du mélange gazeux à mesurer qui dépend de la au moins une concentration du au moins un gaz absorbeur et de la au moins une section transversale effective du au moins un gaz absorbeur, la fonction théorique étant déterminée dans la deuxième étape en spécifiant des valeurs numériques pour la au moins une section transversale effective et le paramètre de dispositif et en utilisant la au moins une concentration comme paramètre à adapter.

6. Procédé selon l'une des revendications précédentes,
**caractérisé en ce que**
une longueur de trajet effective $L_{eff}$ est utilisée comme paramètre d'étalonnage, une longueur de trajet du dispositif est utilisée comme paramètre du dispositif, une longueur de trajet de dispositif $L_0$ est utilisée comme paramètre de dispositif, $L_{eff}$ étant représentée comme produit $L_{eff}(\lambda)=L_0(\lambda)*K(\lambda)$, en particulier $K(\lambda)$ étant représenté comme

$$K(\lambda)=\frac{D_{CE}(\lambda)}{\exp(D_{CE}(\lambda))-1},$$

$D_{CE}$ étant représenté comme

$$D_{CE}(\lambda)=\ln\left[1+L_0(\lambda)\left(\sum_{i=1}^{G}x_i*\sigma_i(\lambda)+f(\vec{m},\lambda)\right)\right],$$

$x_i$ indiquant les valeurs supposées fixes des concentrations des gaz absorbants et $\sigma_i$ indiquant des sections efficaces connues des gaz absorbants en prenant $G$ gaz absorbants différents comme base, $f(\vec{m},\lambda)$ décrivant une atténuation à large bande de la lumière dans la cellule de mesure (3), $\vec{m}$ indiquant une quantité de paramètres d'extinction $m_n$.

7. Procédé selon l'une des revendications précédentes,
**caractérisé en ce que**
au cours de la deuxième étape, dans une première étape intermédiaire, une courbe d'absorption théorique spécifique dépendante de la longueur d'onde est calculée pour chaque gaz absorbeur à partir du paramètre d'étalonnage calculé et d'une section efficace connue au préalable du gaz absorbeur respectif, un spectre d'absorbeur théorique étant calculé par pliage mathématique de la courbe d'absorption respective avec une fonction instrument prédéterminée dans une deuxième étape intermédiaire pour chaque gaz absorbeur respectif, la fonction théorique étant définie en fonction des spectres absorbeurs des gaz absorbeurs respectifs en tant que valeurs numériques dépendant de la longueur d'onde et des concentrations des gaz absorbeurs comme paramètres.

8. Procédé selon la revendication 7,
**caractérisé en ce que**
le paramètre d'étalonnage est calculé par l'intermédiaire de

$$L_{eff}(\lambda) = L_0(\lambda) * K(\lambda) = L_0(\lambda) * \frac{D_{CE}(\lambda)}{\exp(D_{CE}(\lambda)) - 1},$$

où

$$D_{CE}(\lambda) = \ln\left[1 + L_0(\lambda)\left(\sum_{i=1}^{G} x_i \sigma_i(\lambda) + f(\vec{m},\lambda)\right)\right]$$

, où $L_{eff}(\lambda)$ représente le paramètre d'étalonnage, $L_0(\lambda)$ représente le paramètre de dispositif, $\sigma_i$ indique la section transversale effective d'un gaz absorbeur déterminé et $x_i$ la valeur supposée fixe du gaz absorbeur déterminé, en supposant $G$ différents gaz absorbeurs, $f(\vec{m},\lambda)$ indique une atténuation à large bande de la lumière dans la cellule de mesure (3) et $\vec{m}$ indique une quantité de paramètres d'absorption.

9. Procédé selon la revendication 8,
**caractérisé en ce que**
la convolution mathématique pour déterminer le spectre d'absorbeur du gaz absorbeur déterminé est effectuée sur

$$\Theta_i(\lambda) = \frac{1}{x_i}\ln\left[H(\lambda) \otimes e^{-L_{eff}*\sigma_i(\lambda)*x_i}\right]$$

la base de l'équation , $\Theta_i(\lambda)$ indiquant le spectre d'absorbeur du gaz absorbeur déterminé et $H(\lambda)$ indiquant la fonction de l'instrument, la convolution étant effectuée en particulier comme approximation de cette équation avec l'équation de rapprochement $\Theta_i(\lambda) = H(\lambda) \otimes (L_{eff}(\lambda)*\sigma_i(\lambda))$.

10. Procédé selon l'une des revendications précédentes,
**caractérisé en ce que**
on détermine la fréquence à laquelle le cycle est exécuté l'un après l'autre, de la manière suivante

a) un nombre maximum de cycles est spécifié et il est spécifié que lorsque le nombre maximum de cycles est atteint, aucun autre cycle n'est effectué,
b) il est déterminé qu'aucun autre cycle ne doit être effectué si les valeurs supposées pour toutes les concentrations diffèrent de moins d'une valeur limite des valeurs supposées déterminées lors du cycle précédent, et/ou
c) il est déterminé qu'aucun autre cycle n'est effectué dès qu'un résidu entre la fonction de valeur mesurée et la fonction théorique déterminée dans le dernier cycle effectué par la détermination des valeurs supposées est inférieur à une valeur seuil prédéterminée.

11. Procédé selon l'une des revendications précédentes,
**caractérisé en ce que**
la au moins une valeur supposée de la au moins une concentration est fixée à une valeur numérique spécifique dans chaque cas avant l'exécution d'un premier cycle, la valeur numérique spécifique étant lue d'une mémoire du dispositif de mesure (1) ou entrée manuellement par un utilisateur, la valeur mesurée de la concentration spécifique déterminée dans une mesure précédente étant utilisée notamment comme valeur numérique spécifique dans chaque cas.

12. Procédé selon l'une des revendications précédentes,
**caractérisé en ce que**
le mélange gazeux est filtré à travers un filtre aérosol avant il entre dans la cellule de mesure.

13. Dispositif de mesure spectroscopique (1) comprenant une source lumineuse (2), une cellule de mesure (3) avec un résonateur optique, un détecteur (4) et une unité de calcul (5), la source lumineuse (2) étant conçue pour émettre un faisceau lumineux, qui entre dans une cellule de mesure (3) le long d'un trajet de lumière à travers une entrée et sort de la cellule de mesure (3) par une sortie, le détecteur (4) étant disposé à la sortie à l'extérieur de la cellule de mesure (3) et étant conçu pour délivrer des valeurs de mesure dépendant de la longueur d'onde pour une intensité lumineuse de lumière incident sur celui-ci, l'unité de calcul (5) étant conçue pour lire les valeurs mesurées du détecteur (4) et pour représenter un trajet de l'intensité lumineuse dépendant de la longueur d'onde en tant que fonction de valeur mesurée dépendant de la longueur d'onde et pour déterminer la concentration d'au moins un gaz absorbant par un calcul d'adaptation entre une fonction théorique dépendant de la longueur d'onde et la fonction de valeur mesurée dépendant de la longueur d'onde, la fonction théorique contenant un paramètre d'étalonnage dépendant de la longueur d'onde pour tenir compte de l'état du dispositif de mesure (1) et de ladite au moins une concentration dudit au moins un gaz absorbant contenu dans le mélange gazeux comme paramètres à adapter lors du calcul d'adaptation,
**caractérisé en ce que**
l'unité de calcul (5) est adaptée pour effectuer la détermination de la concentration d'au moins un gaz absorbant en définissant le paramètre d'étalonnage en fonction d'un paramètre du dispositif dépendant de la longueur d'onde stocké dans l'unité de calcul (5) caractérisant une longueur de trajet d'un trajet lumineux parcouru par le faisceau lumineux dans la cellule de mesure et un facteur de correction dépendant de la longueur d'onde défini en fonction de ladite au moins une concentration, l'unité de calcul (5) étant conçue pour effectuer un cycle comprenant une séquence de phases, dans laquelle

a) dans une première étape de la séquence, une valeur numérique pour le facteur de correction est calculée à partir d'une valeur supposée fixe pour ladite au moins une concentration via la fonction définissant le facteur de correction,
b) dans une deuxième étape de la séquence, la fonction théorique est déterminée, le paramètre d'étalonnage étant calculé à partir de la valeur numérique pour le facteur de correction calculée dans la première étape,
c) dans une troisième étape de la séquence, un calcul de compensation est effectué au moyen d'une comparaison entre la fonction théorique déterminée dans la deuxième étape et la fonction de la valeur mesurée, dans lequel calcul, à cette fin, ladite au moins une concentration contenue dans la fonction théorique est adaptée comme paramètre librement sélectionnable afin d'adapter la fonction théorique déterminée dans la deuxième étape aussi étroitement que possible à la fonction de la valeur mesurée et une valeur pour ladite au moins une concentration est déterminée et fixée comme nouvelle valeur supposée pour la concentration,

l'unité de calcul (5) étant conçue pour effectuer le cycle plusieurs fois de suite et pour délivrer la au moins une valeur d'acceptation déterminée dans la troisième étape du dernier cycle comme valeur mesurée de la au moins une concentration.

14. Dispositif de mesure spectroscopique (1) selon la revendication 13,
**caractérisé en ce que**
la cellule de mesure (3) est réalisée sous la forme d'une cellule de mesure (3) qui est étanche aux gaz de l'environnement, la cellule de mesure (3) présentant un accès pour l'admission du mélange gazeux dans la cellule de mesure (3), un filtre aérosol pour filtrer les aérosols du mélange gazeux entrant dans la cellule de mesure (3) étant disposé à l'accès.

## Figur 1a

## Figur 1b

## Figur 2

Bestimme die gemessene optische Dichte $M(\lambda) = D_{CE,meas}(\lambda) = \ln\left[\dfrac{I_0(\lambda)}{I(\lambda)}\right]$

Initialisiere $x_i^{(0)}$

Erste Iteration: $\omega = 1$

Berechnung des Korrekturfaktors $K^{(\omega)}(\lambda)$:

$$K^{(\omega)}(\lambda) = \frac{D_{CE}^{(\omega)}(\lambda)}{e^{D_{CE}^{(\omega)}(\lambda)} - 1}$$

mit: $D_{CE}^{(\omega)}(\lambda) = \ln\left[1 + L_0(\lambda)\left(\sum_i \sigma_i(\lambda)\cdot x_i^{(\omega-1)} + \Delta\varepsilon_{Rayleigh}(\lambda)\right)\right]$

Bestimmung der theoretischen Funktion $T^{(\omega)}(\lambda)$:

• Berechnung des Kalibrationsparameters als effektive Weglänge

$$L_{eff}^{(\omega)}(\lambda) = L_0(\lambda)\cdot K^{(\omega)}(\lambda)$$

• Berechnung der Absorberspektren

$$\Theta_i^{(\omega)}(\lambda) = H(\lambda)\otimes\left(L_{eff}^{(\omega)}(\lambda)\cdot\sigma_i(\lambda)\right)$$

$$\rightarrow T^{(\omega)}(\lambda) = D^{(\omega)}{}_{CE,theor.}(\lambda) = \sum_i \Theta_i^{(\omega)}(\lambda) * x_i^{(\omega)} + L_{eff}^{(\omega)}(\lambda) * \Delta\varepsilon_{Rayleigh}(\lambda) + P(\vec{v},\lambda)$$

Ausgleichsrechnung über Anpassung
$T^{(\omega)}(\lambda) = M(\lambda)$ unter Variation von $x_i^{(\omega)}$

Abbruchkriterium erfüllt? —— nein —— $\omega = \omega + 1$

ja

Ende mit finalen Annahmewerten $x_i^{(\omega)}$ als
gemessene Werte der Konzentrationen

# Figur 3

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Nicht-Patentliteratur**

- **D. J. HOCH et al.** An instrument for measurements of BrO with LED-based Cavity-Enhanced Differential Optical Absorption Spectroscopy. *ATMOSPHERIC MEASUREMENT ETCHNIQUES,* 01. Januar 2014, vol. 7 (1), 199-214

- **U. PLATT et al.** Broadband Cavity Enhanced Differential Optical Absorption Spectroscopy (CE-DOAS) - applicability and corrections. *ATMOSPHERIC MEASUREMENT TECHNIQUES,* 16. November 2009, (2), 713-723